(19) Europäisches Patentamt
European Patent Office
Office européen des brevets

(11) Publication number: **0 313 296 B1**

(12) **EUROPEAN PATENT SPECIFICATION**

(45) Date of publication of patent specification: **29.12.93**

(51) Int. Cl.[5]: **C07D 405/12**, C07C 49/755, C07C 49/747, C07D 215/14, C07D 213/30, C07D 215/20, A61K 31/35, A61K 31/47, A61K 31/44

(21) Application number: **88309723.0**

(22) Date of filing: **17.10.88**

# (54) Substituted chromans and related compounds in the treatment of asthma.

(30) Priority: **19.10.87 PCT/US87/02734**

(43) Date of publication of application:
**26.04.89 Bulletin 89/17**

(45) Publication of the grant of the patent:
**29.12.93 Bulletin 93/52**

(84) Designated Contracting States:
**AT BE CH DE ES FR GB GR IT LI LU NL SE**

(56) References cited:
**EP-A- 0 288 962**
**US-A- 4 661 596**

**EUROPEAN JOURNAL OF MEDICINAL CHEMISTRY, vol. 22, 1987, pages 417-419, Elsevier, Paris, FR; R. PERRONE et al.: "Monophenolic 2-amino-1-indanone and 2-amino-1-tetralone derivatives: synthesis and assessment of dopaminergic activity"**

**CANADIAN JOURNAL OF CHEMISTRY, vol. 66, no. 3, 1988, pages 517-526; A. DELGADO et al.: "Synthesis and conformational analysis of 2-amino-1,2,3,4-tetrahydro-1-naphthalenols"**

(73) Proprietor: **PFIZER INC.**
**235 East 42nd Street**
**New York, N.Y. 10017(US)**

(72) Inventor: **Eggler, James Frederick**
**98 Elm Street**
**Stonington, Connecticut(US)**
Inventor: **Melvin, Lawrence Sherman Jr.**
**7 Seabury Avenue**
**Ledyard, Connecticut(US)**
Inventor: **Marfat, Anthony**
**160 Lantern Hill Road**
**Mystic, Connecticut(US)**

(74) Representative: **Bradbrook, Geoffrey William et al**
**PFIZER LIMITED**
**Ramsgate Road**
**Sandwich Kent, CT13 9NJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Background of the Invention

The present invention is directed to substituted tetralins, chromans and related compounds of the formula (I), depicted below, which by inhibiting 5-lipoxygenase enzyme and/or blocking leukotriene receptors, are useful in the prevention or treatment of asthma, arthritis, psoriasis, ulcers, myocardial infarction and related disease states in mammals. The present invention is also directed to pharmaceutical compositions, a method of treatment and to intermediates useful in the synthesis of said compounds of the formula (I).

Kreft et al., in U.S. Patent 4,661,596, describe compounds which are disubstituted naphthalenes, dihydronaphthalenes or tetralins having the formula

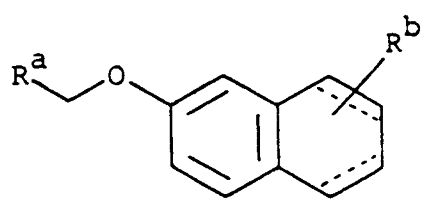

wherein the dotted lines represent optional double bonds, $R^a$ is 2-pyridyl, 2-quinolyl, 2-pyrazinyl, 2-quinoxalinyl, 2-thiazolyl, 2-benzothiazolyl, 2-oxazolyl, 2-benzoxazolyl, 1-alkyl-2-imidazolyl or 1-alkyl-2-benzimidazolyl and $R^b$ is hydroxy, lower alkoxy, lower alkyl or perfluoro alkyl. Like the compounds of the present invention, these compounds inhibit lipoxygenase enzyme and antagonize the effects of leukotriene D4, and so are useful in the prevention and treatment of asthma.

The chemical nomenclature employed herein generally follows that of "I.U.P.A.C. Nomenclature of Organic Chemistry, 1979 Edition," Pergammon Press, New York, 1979.

### Summary of the Invention

The present invention is directed to racemic or optically active compounds having the structural formula

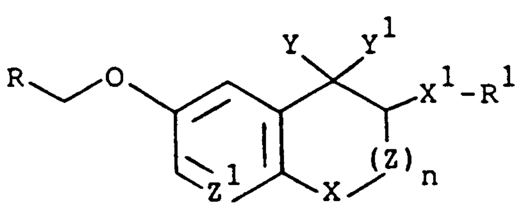

---(I)

wherein

n is 0 or 1;

X is O, S, SO, $SO_2$, NH or $N(C_1\text{-}C_4)$alkyl;

$X^1$ is $CH_2$, O, S, SO or $SO_2$;

Y and $Y^1$ are taken together and form a carbonyl group, or Y and $Y^1$ are taken separately, Y is hydrogen and $Y^1$ is hydroxy or an acyloxy group which is hydrolyzed to form a hydroxy group under physiological conditions;

Z is $CH_2$, $CHCH_3$, $CH_2CH_2$ or $CH_2CH_2CH_2$;

$Z^1$ is CH or N;

R is 2-, 3- or 4-pyridyl, 2-, 3- or 4-quinolyl, 1-, 3- or 4-isoquinolyl, 3- or 4-pyridazinyl, 3- or 4-cinnolinyl, 1-phthalazinyl, 2- or 4-pyrimidinyl, 2- or 4-quinazolinyl, 2-pyrazinyl, 2-quinoxalinyl, 1-, 2- or 3-indolizinyl, 2-, 4- or 5-oxazolyl, 2-benzoxazolyl, 3-, 4- or 5-isoxazolyl, 5-benzo[c]isoxazolyl, 3-benzo[d]isoxazolyl, 2-, 4- or 5-thiazolyl, 2-benzothiazolyl, 3-, 4- or 5-isothiazolyl, 5-benzo[c]isothiazolyl, 3-benzo[d]isothiazolyl, 1-[($C_1$-$C_4$)alkyl]-2-, 4- or 5-imidazolyl, 1-[($C_1$-$C_4$)alkyl]-2-benzimidazolyl, 1-[($C_1$-$C_4$)alkyl]-3-, 4- or 5-pyrazolyl, 2-[($C_1$-$C_4$)alkyl]-3(2H)-indazolyl, or 1-[($C_1$-$C_4$)alkyl]-3(1H)-indazolyl; or one of said groups mono- or disubstituted on carbon with the same or different substituents which are bromo, chloro, fluoro, ($C_1$-$C_4$)alkyl,

trifluoromethyl, hydroxy, hydroxymethyl or $(C_1-C_4)$alkoxy, or substituted on adjacent carbons with trimethylene, tetramethylene, $-CH_2-O-CH_2-$ or $-O-CH_2-O-$; and

$R^1$ is $(C_1-C_8)$alkyl, $(C_3-C_8)$cycloalkyl, $(C_7-C_{10})$bicycloalkyl, $(C_4-C_{10})$cycloalkylalkyl, $(C_8-C_{11})$-bicycloalkylalkyl, or one of said groups mono- or disubstituted with the same or different groups which are fluoro, $(C_1-C_4)$alkyl, $(C_1-C_4)$alkoxy, carboxy, $[(C_1-C_4)$alkoxy]carbonyl, or $(C_2-C_5)$alkanoyl;

a pharmaceutically acceptable acid addition salt thereof; or

a pharmaceutically acceptable cationic salt when the compound contains a carboxy group.

Because of their ease of preparation and valuable biological activity, the preferred compounds of the formula (I), regardless of the value of Y and $Y^1$, have n as 1, Z as $CH_2$, $Z^1$ as CH, X as O and $X^1$ as $CH_2$ or O. More preferred compounds further have R as 2-pyridyl or 2-quinolyl and $R^1$ as $(C_2-C_8)$alkyl or $(C_3-C_8)$-cycloalkyl. Most preferred are racemic or optically active compounds having the relative stereochemical formula

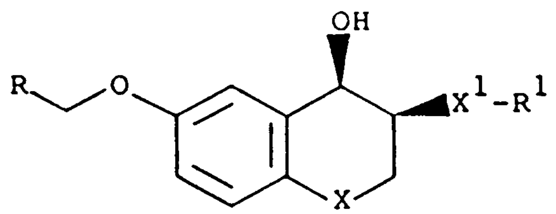

---(II)

or

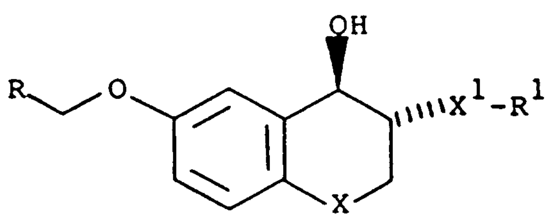

, ---(III)

most particularly those racemic or optically active compounds of the formula (II) or (III) wherein X and $X^1$ are each O, R is 2-quinolyl, and $R^1$ is isopropyl or cyclohexyl;

Said pharmaceutically-acceptable acid addition salts include, but are not limited to, those with HCl, HBr, $HNO_3$, $H_2SO_4$, $H_3PO_4$, methanesulfonic acid, p-toluenesulfonic acid, maleic acid and succinic acid. In the case of those compounds of the formula (I) which contain a further basic nitrogen, it will, of course, be possible to form diacid addition salts (e.g., the dihydrochloride) as well as the usual monoacid addition salt. Said pharmaceutically-acceptable cationic salts include, but are not limited to, those of sodium, potassium, calcium, magnesium, ammonia, N,N'-dibenzylethylenediamine, N-methylglucamine (meglumine), ethanolamine and diethanolamine.

The reference to $Y^1$ as an acyloxy group which is hydrolyzed to a hydroxy group under physiological conditions refers to esters of a type which are frequently referred to as "pro-drugs." Such esters are now as well-known and common in the medicinal art as pharmaceutically-acceptable salts. Such esters are generally used to enhance oral absorption, but in any event are readily hydrolyzed in vivo to the parent hydroxy compound. The more preferred acyloxy groups are those in which the acyl moiety is

the alpha-aminoacyl residue of a naturally occurring L-alpha-amino acid,

$$-\overset{O}{\overset{\|}{C}}-(CH_2)_pNR^2R^3,\quad -\overset{O}{\overset{\|}{C}}-CHNH_2(CH_2)_qNR^2R^3,$$

$$-\overset{O}{\overset{\|}{C}}-(CH_2)_rCOOH,\ \text{or}\ -\overset{O}{\overset{\|}{C}}-CHNH_2(CH_2)_sCOOH;$$

wherein

$R^2$ and $R^3$ are taken separately and are each independently hydrogen or ($C_1$-$C_4$)alkyl, or $R^2$ and $R^3$ are taken together with the nitrogen to which they are attached to form a pyrrolidine, piperidine, perhydroazepin or morpholine ring;

p is an integer from 1 to 4;

g is an integer from 1 to 3;

r is an integer from 2 to 3; and

s is an integer from 1 to 3.

Also forming a part of the present invention are pharmaceutical compositions for administration to a mammal which comprise a compound of the formula (I) and a pharmaceutically acceptable carrier; and a method of inhibiting 5-lipoxygenase enzyme and/or blocking leukotriene D4 receptors in a mammal, so as to prevent or treat asthma (particularly in man), arthritis, psoriasis, gastrointestinal ulcers, or myocardial infarction.

Finally, the present invention is directed to valuable intermediate compounds having the structural formula

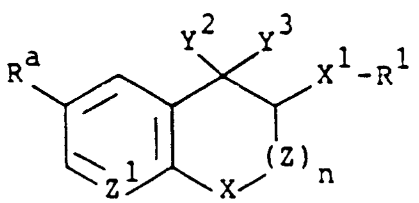

---(IV)

wherein

n, X, Z and $Z^1$ are as defined above;

$Y^2$ and $Y^3$ are taken together and form a carbonyl group, or $Y^2$ and $Y^3$ are taken separately, $Y^2$ is hydrogen and $Y^3$ is hydroxy; and

$R^a$ is hydroxy or benzyloxy.

Detailed Description of the Invention

The present invention is readily carried out. Without regard to geometrical (cis-trans) or optical isomers, the compounds of the formula (I) wherein Y + $Y^1$ = carbonyl, or Y = H and $Y^1$ = OH, and $X^1$ = $CH_2$, S or O are prepared according to the chemical transformations which are summarized in Flowsheets 1, 2 and 3, where the symbols n, X, Z, $Z^1$, R and $R^1$ are as defined above. The various transformations found in these flowsheets, as well as transformations required for the preparation of the compounds (I) having other values of Y, $Y^1$ and $X^1$, and methods for separation of cis-trans and optical isomers, are detailed below.

The condensation of Flowsheet 1 is typically carried out with the phenolic group in protected form as shown, methyl being a preferred protecting group only when $X^1$ is $CH_2$. The preferred conditions employ a molar excess of the required aldehyde and a molar excess of a secondary amine such as pyrrolidine or piperidine as base. (It is understood that such a base facilitates the condensation by forming an enamine intermediate.)

Flowsheet 1

When $X^1 = CH_2$

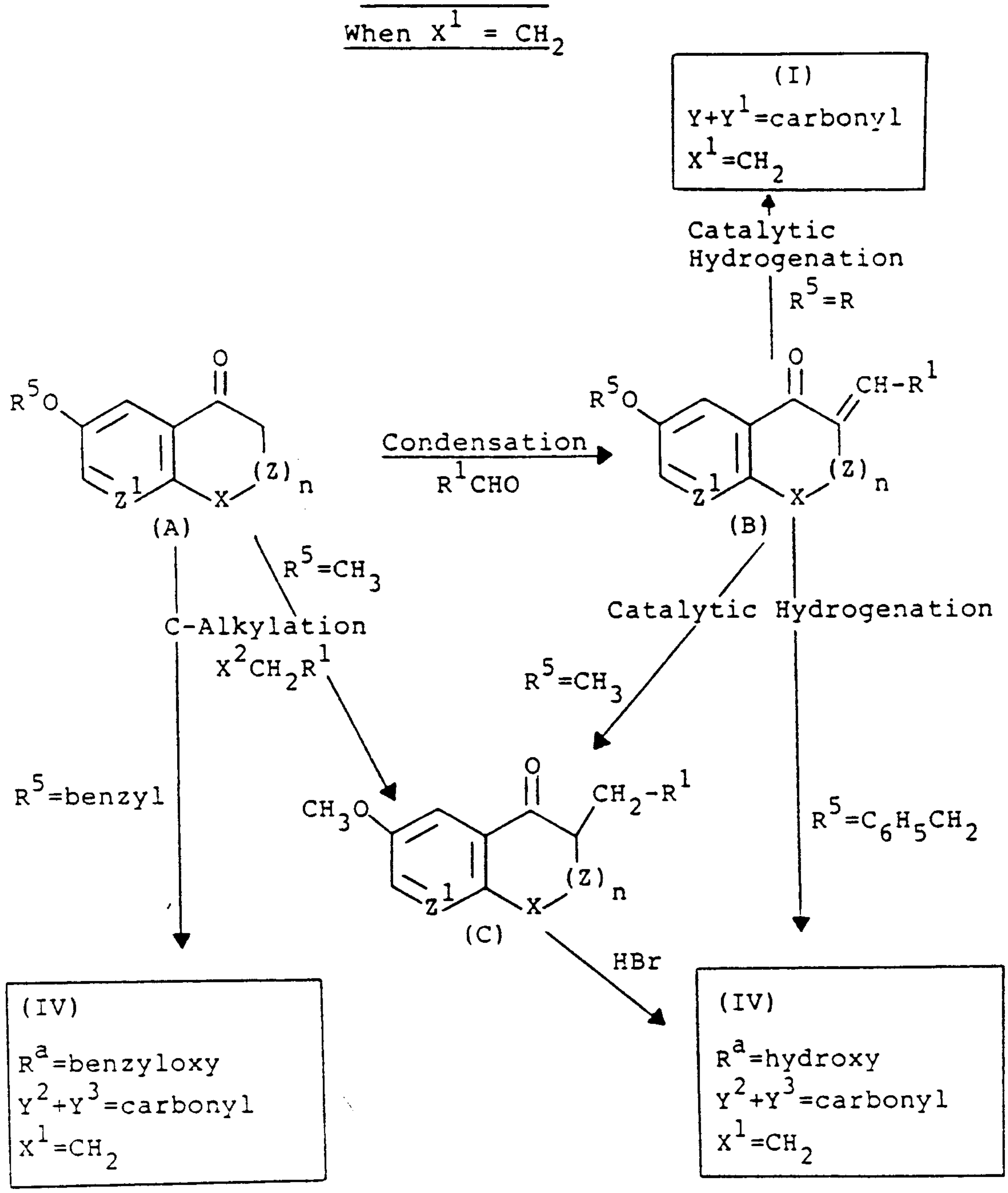


$R^5$=R, $CH_3$, or $C_6H_5CH_2$

$X^2$=Nucleophically displaceable group such as I, Br, Cl, $CH_3SO_3$ or p-$CH_3C_6H_4SO_3$

Flowsheet 2

When $X^1$ = O or S

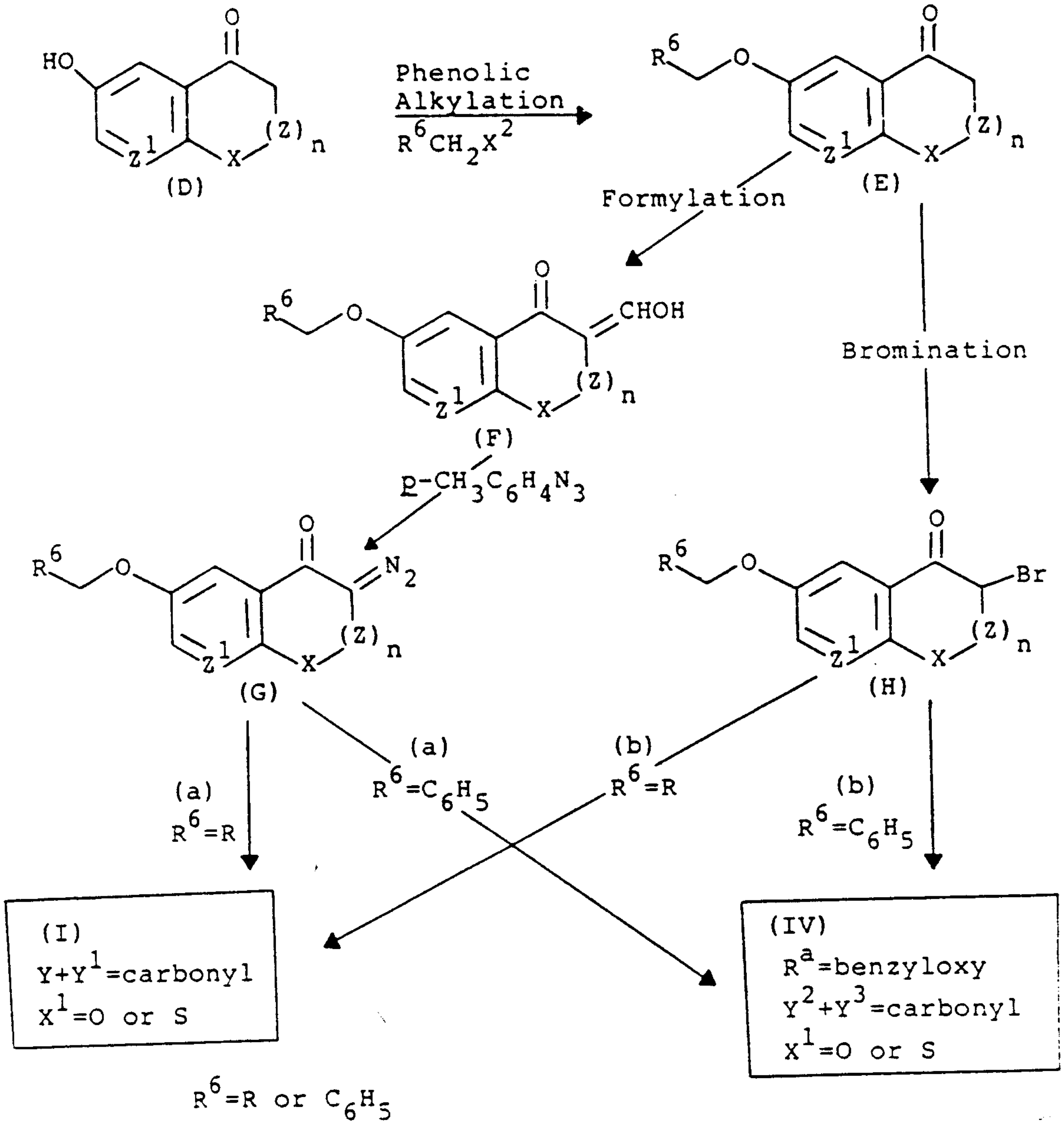


$R^6$=R or $C_6H_5$

$X^2$=Cl, Br, I, $CH_3SO_3$, p-$CH_3C_8H_4SO_3$ or other nucleophilically displaceable group

(a) $R^1SH$ or $R^1OH$, rhodium (II) acetate dimer

(b) $R^1SH$ or $R^1OH$, base

Flowsheet 3

When $X^1$ = $CH_2$, O or S

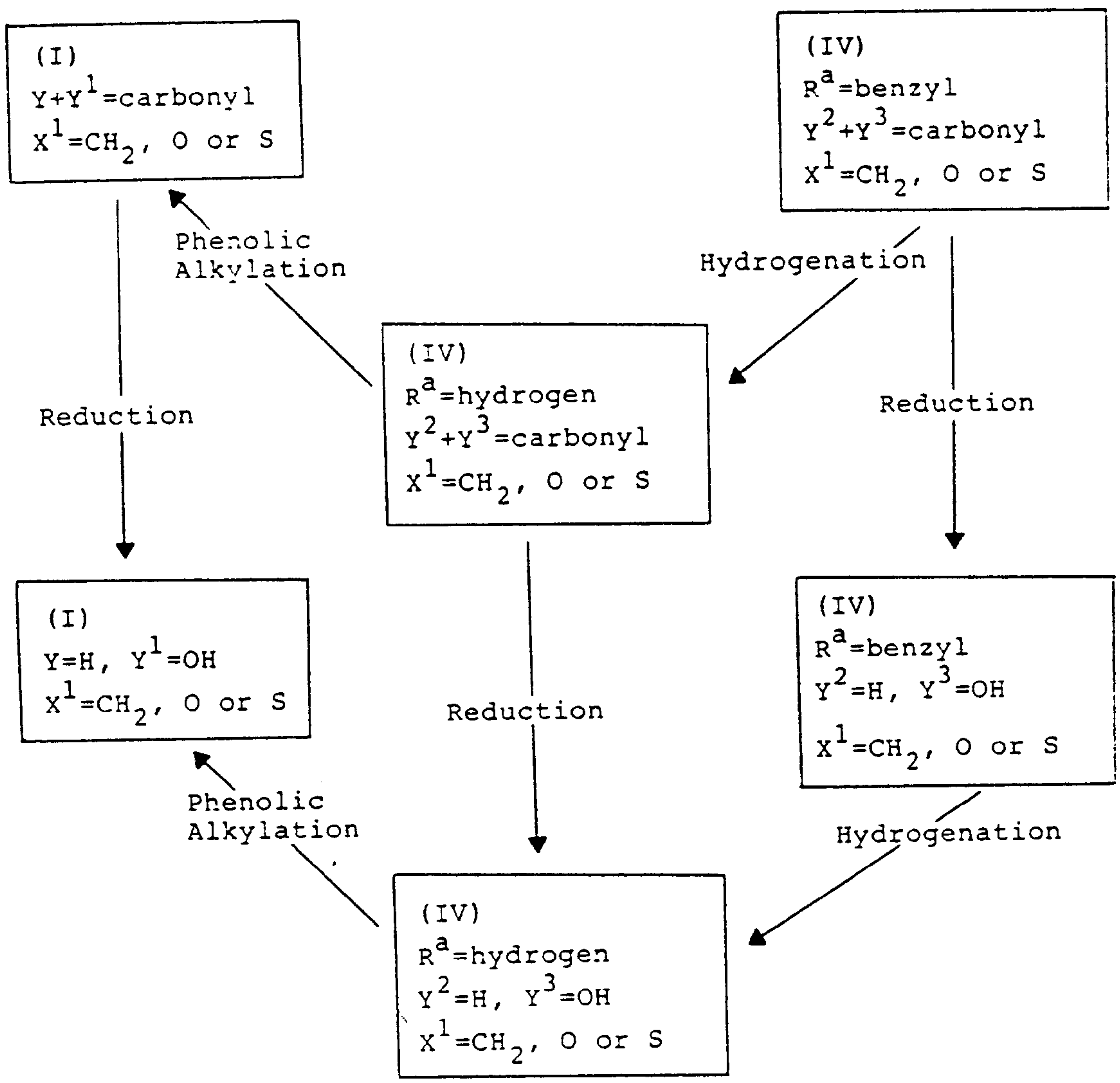


The reaction is generally carried out in a reaction-inert solvent, lower alcohols such as methanol being particularly well suited for this purpose. The temperature conditions for this transformation are not critical, e.g., 0-70° C. is generally satisfactory, with ambient temperature particularly well suited as a matter of convenience.

As used here and elsewhere herein, the expression "reaction-inert solvent" refers to a solvent which does not interact with starting materials, reagents, intermediates or products in a manner which adversely affects the yield of the desired product.

The C-alkylation of Flowsheet 1 is carried out by first converting the ketone (A) to its lithium salt, usually in situ, by the action of substantially one molar equivalent of a strong, sterically hindered base such as lithium diisopropylamide, usually carried out at low temperature (e.g., about -40 to -80° C. conveniently at the temperature of a dry ice-acetone bath). The salt in turn is reacted with the alkylating agent, preferably the highly reactive iodide, usually in molar excess in the presence of a molar excess of hexamethyl phosphoramide, now at higher temperature (e.g., about 0 to 40° C.). Conveniently, the latter reagents are added to the cold lithium salt solution, and the temperature allowed to rise to ambient temperature as the reaction proceeds. The salt preparation and alkylation reaction are usually carried out in the same reaction-inert solvent (e.g., tetrahydrofuran). It will be evident to those skilled in the art that any free hydroxy or

carboxy groups in the alkylating reagent should be in protected form (vide supra).

The catalytic hydrogenation transformations (debenzylations, $H_2$-additions to double bond) of Flowsheets 1, 2 and 3 are carried out under conventional conditions, generally in a reaction-inert solvent, and preferably using a noble metal catalyst and moderate conditions of temperature (e.g., about 0 to 70° C.) and hydrogen pressure (e.g., about 1 to 10 atmospheres). While higher pressures may be desirable in selected instances, such moderate pressures permit the use of much less elaborate and expensive equipment. Suitable noble metal catalysts include platinum, palladium, rhenium, rhodium and ruthenium, either of the supported or non-supported type, as well as the known catalytic compounds thereof such as the oxides, chlorides, etc. Examples of suitable catalyst supports include carbon, silica and barium sulfate. The catalysts may be preformed or formed in situ by prereduction of an appropriate salt of the catalytic compound. Examples of preferred catalysts are 5% palladium-on-carbon, 5% platinum-on-carbon; 5% rhodium-on-carbon, platinum chloride, palladium chloride, platinum oxide and ruthenium oxide. Most preferred in the present instance is palladium-on-carbon. Solvents generally suitable for the present hydrogenation include lower alkanols, ethyl acetate and tetrahydrofuran.

The methyl ethers [compounds of the formula (C)] in Flowsheet 1 are deblocked to form the corresponding phenol derivative, again, by conventional methods; for example, using of concentrated HBr, or $BBr_3$, both of which are exemplified below.

The phenolic alkylations found in Flowsheets 2 and 3 and the bromine replacement reaction of Flowsheet 2 each represent conventional nucleophilic displacement reactions. These displacements are generally carried out in the presence of a base of sufficient strength to convert the displacing phenol, alcohol or thiol to its salt, and in a quantity at least sufficient to neutralize the by-product acid ($HX^2$, HBr). In those substrates which contain an aliphatic alcohol group [e.g., a compound (IV) wherein $Y^2$ is H and $Y^3$ is OH], bases of sufficient strength to convert that group to the anion will generally be used in an amount no more than sufficient to convert the more acidic phenol to the salt. When either of the reactants contains a group of acidity similar to or greater than that of the nucleophilic displacing compound, such potentially interfering groups are best introduced in protected form (e.g., a heteroaromatic phenolic group as methoxy or benzyloxy, a carboxy group as methyl or benzyl ester, removable by hydrolysis or hydrogenolysis according to methods detailed elsewhere herein). The present nucleophilic displacements are carried out in a reaction-inert solvent, preferably, one which is much less acidic than the displacing phenol, alcohol or mercaptan. Most preferred are polar, aprotic solvents such as dimethylformamide or acetone, usually with a molar excess of the more readily available of the two reactants. Temperature is not critical, e.g., about 10-70° C. is usually satisfactory with ambient temperature most convenient. In one preferred variant, the phenol, alcohol or mercaptan is irreversibly converted to the anion with a base such as sodium hydride. Other preferred variants employ $K_2CO_3$ as base in the presence of NaI, or $Cs_2CO_3$ as base in the presence of CsI.

In the special case of X = NH, such nucleophilic displacements will generally be carried out with the NH group protected, e.g., as the N-benzyl derivative (subsequently removed by hydrogenation) or as an N-alkanoyl or N-sulfonyl derivative (subsequently removed under appropriate hydrolysis conditions; for example, the N-tosyl derivative is hydrolyzed by heating in a mixture of acetic acid and concentrated HCl).

The formylation of Flowsheet 2 represents a conventional condensation type reaction of a ketone with an alkyl formate. This reaction is generally in an aprotic reaction-inert solvent such as toluene in the presence of a strong base such as sodium hydride at moderate temperatures (e.g., 0-70° C., conveniently at ambient temperature). The subsequent conversion to the diazo compound is conveniently accomplished with tosyl azide as the reagent, a reaction generally carried out at low temperature (e.g., about -10 to -60° C.) in the presence of molar excess of a tertiary amine (e.g., triethylamine) in a reaction-inert solvent such as $CH_2Cl_2$. In turn, the diazo compound is reacted with an appropriate alcohol or mercaptan in the presence of a catalytic amount of rhodium (II) diacetate dimer to form the desired ether or thioether. The latter transformation is generally carried out in an anhydrous reaction-inert solvent such as toluene at somewhat elevated temperature, e.g., about 500-100° C. Substituent alcohol or carboxy groups which are not intended to react are preferably protected in this transformation, as in the case of the nucleophilic displacement reactions discussed above.

The "reduction" reactions of Flowsheet 3 require the reduction of a ketone to a secondary alcohol, for which a number of selective reagents are available. Where no other $LiAlH_4$ reducible groups (such as carboxy, methoxycarbonyl) are present, that reagent is well suited for this purpose. On the other hand, $NaBH_4$ is preferred as the reducing agent when such reducible groups are present. In either case, these hydride reductions are generally carried out in a reaction-inert solvent (such as tetrahydrofuran in the case of $LiAlH_4$, methanol or a methanol/tetrahydrofuran combination in the case of $NaBH_4$). In either case, temperature is not critical, about 0 to 50° C. being generally satisfactory and ambient temperature

preferred. The present reduction step offers the potential of producing a mixture of cis- and trans-isomers [as illustrated in the formulas (II) and (III)] and in the present hydride reduction, that is the result which is generally observed. If one or the other of these isomers is particularly desired, one can usually find a reduction method and set of conditions which will favor the desired isomer. For example, $NaBH_4$ reduction in the presence of cesium chloride will generally strongly favor the cis-isomer. Catalytic hydrogenation is also a generally useful reduction method, generally carried out under conditions which are somewhat more vigorous than those described above (e.g., more prolonged time, higher catalyst level, higher temperature and/or higher pressure). Hydrogenation is preferably carried out on substrates such as

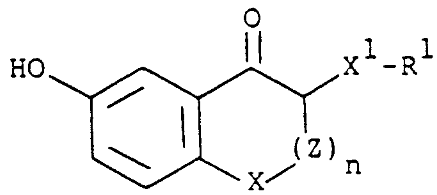

---(V)

which contain no other readily hydrogenated group. Pd/C catalyst tends to particularly favor formation of cis-isomer. However, by variation of the catalyst and conditions, it will be possible to modify or even reverse that tendency. Where both cis- and trans-isomers form in the present reduction, they are generally separable by standard chemical methods (e.g., selective or fractional crystallization, chromatography, and so forth).

If compounds wherein $X^1$ is SO or $SO_2$ are desired, they are usually prepared from the corresponding compounds of the formula (I) or (IV) wherein the group $X^1$ as S is already in place. Peroxides are generally used as oxidizing agent. A particularly convenient reagent for this purpose is m-chloroperbenzoic acid. The sulfide is reacted with substantially 1 molar equivalent of this reagent to obtain the sulfoxide and with at least 2 molar equivalents to obtain the sulfone, in a reaction-inert solvent such as $CH_2Cl_2$. Temperature is not critical, e.g., 0-60° C. being generally satisfactory and ambient temperature preferred. However, when X is S, and compounds wherein $X^1$ is SO or $SO_2$ are desired, these are preferably formed by conventional sulfinylation of sulfonylation of an unsubstituted ketone compound of the formula (A), (D) or (E).

Those ketone compounds of the formula (I) or (IV) wherein Y and $Y^1$ or $Y^2$ and $Y^3$ form a carbonyl group contain an asymmetric carbon at the alpha-position which is adjacent to the carbonyl group, and therefore are racemic compounds capable of resolution into optically active enantiomers, e.g., by conversion of the racemate into diastereomeric salts with an optically active acid, which are generally separable by a fractional crystallization process. Alternatively, if the substrate contains a carboxy group, separable diastereomeric salts are formed with an optically active organic amine. Optical activity can also be induced by use of an optically active reagent in the step by which the asymmetric carbon is formed, e.g., use of an optically active Wilkinson type catalyst, or a noble metal supported on an optically active support, in the hydrogenation step. The optically active ketones are also available by conventional reoxidation of an optically active alcohol of the next paragraph, e.g., via the Jones oxidation, which is exemplified below.

The hydroxy compounds of the formula (I) and (IV) wherein Y (or $Y^2$) is hydrogen and $Y^1$ (or $Y^3$) is OH contain two such asymmetric carbons--corresponding to two racemates and four optically active compounds. One of these racemates is the above noted cis-isomer, and the other the trans-isomer. Each of these racemates is capable of resolution into a pair of enantiomers via diastereomeric salts, as detailed in the preceding paragraph. It is preferred, however, to convert the racemic alcohol to corresponding diastereomeric esters or urethanes formed with an optically active acid or isocyanate. Such covalently bonded derivatives are generally subjectable to a broader variety of separation methods (e.g., chromatography) than are diastereomeric salts. Such diastereomeric esters are formed from the alcohol and the optically active acid by standard methods, generally those involving activation of the acid, e.g., as the acid chloride, as a mixed anhydride with an alkyl chloroformate, or with a dehydrative coupling agent such as dicyclohexylcarbodiimide. A preferred optically active acid in the present case is S-O-acetylmandelic acid. Once the resulting diastereomeric esters are separated, e.g., by chromatographic methods, they are hydrolyzed by conventional methods, e.g., aqueous acid or aqueous base, to obtain the enantiomeric, optically active alcohols.

The prodrug esters of the present invention are prepared by methods similar to those used in the synthesis of esters in the preceding paragraph. Esters with alpha-amino acids, including natural L-amino acids, will generally by prepared from the appropriate amino acid in which the alpha-amino group,

substituent $NH_2$ or NH groups (e.g., lysine, ornithine, arginine, histidine, tryptophan), hydroxy groups (serine, homoserine, threonine, tyrosine), mercapto groups (cysteine) and substituent carboxy groups (glutamic acid, aspartic acid) are in protected form (e.g., N-benzyloxycarbonyl, O- and S-benzyl) generally removed by catalytic hydrogenation in a subsequent step. Similarly, in the case of esters with primary or secondary amino substituents, the acids will be coupled with amino groups protected. Such protection is, of course, unnecessary with those acids containing tertiary amino substituents. Finally, the carboxy substituted esters are most conveniently prepared from the cyclic anhydride:

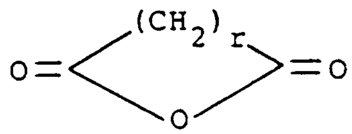

.

Concerning the biological activity of the present compounds, it is known that arachidonic acid is metabolized in mammals by means of two distinct pathways, one leading to prostaglandins and thromboxanes, the other to several oxidative products called leukotrienes, which are designated by letter number combinations such as B4, C4 and D4. The first step in this oxidative pathway is the oxidation of arachidonic acid under the influence of 5-lipoxygenase enzyme, an enzyme which is generally inhibited by the compounds (I) of the present invention, thus blocking the synthesis of all leukotrienes. That in itself provides the mechanism sufficient for the utility of the present compounds in the treatment or prevention of asthma (where LTC4 and LTD4 are understood to be mediators), arthritis (where LTB4 is understood to be a mediator in inflammation), psoriasis (where LTB4 is understood to be a mediator), ulcers (where LTC4 and LTD4 are understood to be mediators) and myocardial infarction (where LTB4 is understood to be a mediator). Supplementing this enzyme inhibitory activity is the general ability of the present compounds to antagonize leukotriene D4 (i.e., block LTD4 receptors). In general, the present compounds also antagonize leukotriene B4. For a review concerning leukotrienes, see Bailey et al., Ann. Reports Med. Chem. 17, pp. 203-217 (1982).

The in vitro activity of the compounds of the formula (I) is tested as follows. RBL-1 cells, maintained in monolayer form are grown for 1 or 2 days in spinner culture in Minimum Essential Medium (Eagle) with Earl's Salts plus 15% Fetal Bovine Serum supplemented with antibiotic/antimycotic solution (GIBCO). The cells are washed 1 time with RPMI 1640 (GIBCO) and resuspended in RPMI 1640 plus 1 microM glutathione to a cell density of 1 x 10 cells/ml. A volume of 0.5 ml of the cell suspension is incubated at 30° C. with 0.001 ml of dimethylsulfoxide solution of drug for 10 minutes. The reaction is started by a simultaneous addition of 0.005 ml (14C)-arachidonic acid in ethanol and 0.002 ml A23187 in dimethylsulfoxide to give final concentrations of 5.0 and 7.6 microM, respectively. After a 5 minute incubation at 30° C., the reaction is stopped by the addition of 0.27 ml acetonitrile/acetic acid (100/0.3) and the media is clarified by centrifugation. Analysis of the product profile is made by a 0.2 ml injection of the clarified supernatant into HPLC. The separation of radioactive products is effected on a radial PAX CN column (5 mm I.D., Waters) with a solvent system of acetonitrile/$H_2O$/acetic acid (0.1%) with a linear acetonitrile gradient from 35% to 70% over 15 minutes at 1 ml/minute. Quantitation is accomplished with a Berthold Radioactivity Monitor equipped with a built-in integrator and a 0.2 ml flow cell mixing 2.4 ml/minute Omnifluor (NEN) with column effluent. Integration units for each product are calculated as a percentage of total integration units, and then compared to the average control levels. The results are expressed as "Percent of Control" and are plotted vs the log of drug concentration. The $IC_{50}$ values are estimated by graphical inspection.

The leukotriene D4 (LTD4) receptor assay tests the ability of a compound to compete with radiolabelled LTD4 for specific LTD4 receptor sites on guinea pig lung membranes. In this test, normal 3-4 week-old guinea pigs are acclimatized under standard conditions for 3 days prior to being sacrificed. Final animal age: 24-31 days. The guinea pigs are stunned by a blow to the back of the neck, and exsanguinated by cutting the carotid artery. The chest cavity is opened and the lungs are removed, rinsed in 50 mM Tris buffer (pH 7.0) and placed in clean buffer. In this and all subsequent operations, all tissue and buffer are kept on ice throughout the preparation, and all centrifugation is carried out at 4° C. Bronchi and connective tissue are trimmed from the lungs. The tissue is weighed and placed in 50 ml polycarbonate tubes with buffer at a ratio of 1 gm tissue/3 ml buffer. The tissue is homogenized by a Tekmar Tissumizer at full speed for 30 seconds and centrifuged in a Sovall SS-34 rotor at 3250 rpm x 15 minutes. The supernatant is centrifuged at 19,000 rpm x 10 minutes. The resulting pellet is resuspended in buffer with the Tissumizer at medium speed (position 75) for 10 seconds. The resuspension is again centrifuged at 19,000 rpm x 10 minutes. The resulting pellet is resuspended by the Tissumizer at slow speed (position 50) for 10 seconds

in 1 ml buffer/g of starting tissue. This final suspension is stirred at 4° C. while aliquoted to polypropylene tubes and stored at -70° C. The following are added to a 12x75 mm polystyrene tube:

(1) 25 microL of one of the following:

A. Dimethylsulfoxide (to determine total binding)

B. 1 microM LTD4 (to determine non-specific binding)

C. 30 nanoM - 100 microM compound in dimethylsulfoxide

(2) 0.025 ml 3H-LTD4 (specific activity 30-60 Ci/mmol) in 50 mM Tris (pH 7.0) + 10 microM L-cysteine (12,000 - 15,000 cpm/0.025 ml)

(3) 0.2 ml diluted membrane preparation (1 mg/ml) (The preparation is diluted in 50 microM Tris buffer + $MgCl_2$ such that in 200 microL protein, a 10 microM $MgCl_2$ concentration is achieved).

The reaction tubes are incubated at 25° C. for 30 minutes. Four ml of cold Tris buffer + 10 microM $MgCl_2$ are added to each tube. The contents are quickly filtered through a Whatman GF/C filter with a Yeda separation device. The filter is washed 3X with 4 ml Tris-$MgCl_2$ buffer. The filter is transferred to a scintillation vial. Ultrafluor scintillation fluid is added. The vial is capped, vortexed and counted for 3 hours. Percent specific binding is calculated using the formula:

% SB = (X - NSB)/(TB - NSB) ,

where

X = cpm sample

NSB = cpm non-specific binding

TB = cpm total binding

Percent specific binding is graphed as a function of compound concentration. $IC_{50}$ is that concentration at which 50% SB occurs. Ki is calculated by using the formula:

Ki = ($IC_{50}$)/[1 + (L/Kd)] ,

where

L = concentration of ligand added (microM) = cpm added/cpm of 1 microM 3H-LTD4

Kd = 1 microM (dissociation constant)

Human polymorphonuclear leukocytes are employed to measure the competition of test molecules with [3H]-LTB4 for binding at the LTB4 receptor. In this test neutrophils are isolated from heparinized human peripheral blood (usually 100 ml) using a Hypaque-Ficoll gradient (density 1.095 g/ml). Hanks balanced salt solution (HBSS) containing 0.1 grams/100 ml bovine serum albumin (HBSS-BSA) is used to resuspend the cells. The one step Hypaque-Ficoll technique yields highly pure populations of neutrophils (greater than 95%). Cell viability is assessed by trypan blue dye exclusion (should be greater than 95%), and the functional integrity of the neutrophils was determined by nitroblue tetrazolium reduction (should be greater than 85% positive). Compounds undergoing test are dissolved in dimethylsulfoxide at a concentration of 100 microM. These solutions are diluted by a factor of 500 using HBSS-BSA. A concentration of 100 microM drug is achieved by introducing the diluted sample in a 0.5 ml aliquot into the reaction tube. Serial dilutions of 1-3 and 1-5 are made (as appropriate) and a 0.5 ml aliquot of these dilutions is added to the incubation tube. [3H]-LTB4 (NEN:specific radioactivity, greater than 180 Ci/mmol; 0.005 ml in absolute ethanol) is introduced into borosilicate tubes (12 x 75 mm). A volume of 0.5 ml of the drug solution (see above) is then added. The binding reaction is initiated by adding 0.5 ml of ice cold neutrophils at a cell density of [5 x $10^6$ cells/ml], and continued at 4° C. for 30 minutes. The incubation is terminated by rapid filtration through a Whatman GF/C glass filter to separate the free from the bound radiolabelled ligand. The filters are washed 3-times with 3 ml ice-cold HBSS, dried, placed in 4 ml of Ultrafluor, and counted. Total binding is defined as the CPM present on the filter (cell associated) when radiolabelled ligand is incubated with neutrophils in the absence of any competing agent. Nonspecific binding is obtained by incubating cells with radiolabelled ligand plus 1 microM nonradiolabelled LTB4. Specific binding is total binding CPM corrected for the nonspecific binding CPM. Every tube is corrected for nonspecific binding. Points of half-maximal displacement of radiolabelled ligand are estimated by graphical analysis on a semi-logarithmic plot of percent of specific binding (no competitor present) vs concentration.

To evaluate the compounds of the formula (I) in vivo, they are tested by the so-called PAF lethality assay procedure:

Materials:

| | |
|---|---|
| Mice: | CD1 males, all approximately the same weight (approximately 26 grams), 12 per group. |
| Vehicle for oral drug dosing: | EES (5% ethanol, 5% emulphor, 90% saline). Stored at room temperature. |
| Drugs: | For routine screening at 50 mg/kg, 20 mg drug is dissolved in 4 ml EES, using sonication in a sonicator bath or grinding in a Ten Broeck grinder to dissolve drug if necessary. If solubility is still a problem, the drug is used as a suspension. |
| Vehicle for i.v. Injection: | Saline with 2.5 mg/ml Bovine Serum Albumin (BSA, Sigma #A4378) and 0.05 mg/ml Propranolol (Sigma #P0884). Prepared fresh daily and kept at room temperature. |
| Platelet Activating Factor (PAF): | A 10 microM stock solution is prepared by dissolving 1 mg PAF (Calbiochem #429460) in 0.18 ml ethanol. This is stored at -20° C. and is diluted in vehicle (see above) the day of use. The concentration of PAF used is calibrated so that when injected at 0.1 ml/10 grams body weight, it will kill approximately 80% of untreated controls. This is usually about 0.028 g/kg (a 1 to 2034 dilution from stock). The solution is prepared in glass containers and is used with glass syringes to minimize surface adhesion by the PAF. It is kept at room temperature. |
| Positive Control: | Phenidone is used at 25 mg/kg (its approximate ED 50). |

Method:

45 minutes before PAF injection, mice are treated orally with drug using 0.1 ml/10 grams body weight. 35 to 40 minutes later they are placed under a heat lamp to dilate the caudal vein for PAF injection. PAF is injected i.v. at 0.1 ml/10 grams body weight, and death follows usually within 30 minutes, rarely after 60 minutes. Results are expressed as percent mortality as compared to controls. Because the assay appears to be sensitive to endogenous catecholamines (i.e., beta agonists protect the mice), Propranolol is used to overcome this potential problem. It also helps if the mice are acclimated to the room before testing, and if room noise and temperature are kept moderate and constant. The heat lamp distance should be calibrated so as to permit vasodilation without visible stress to the mice. Fasting the mice should be avoided.

Variations:

1. The time for oral dosing can be changed.
2. Intravenous drug dosing is possible by coinjecting the drug with PAF in the same volume and vehicle as described above. For coinjection, PAF is prepared at twice the desired concentration in saline with BSA and Propranolol as above, and the drug is prepared at twice the desired concentration in the same vehicle. The two preparations are mixed in equal volumes immediately before injection.

For use in the prevention or treatment of asthma, arthritis, psoriasis and gastrointestinal ulcers in a mammal, including man, a compound of the formula (I) is given in a 5-lipoxygenase inhibiting and/or leukotriene receptor blocking amount of about 0.5-50 mg/kg/day, in single or divided daily doses. A more preferred dosage range is 2-20 mg/kg/day, although in particular cases, at the discretion of the attending physician, doses outside the broader range may be required. The preferred route of administration is generally oral, but parenteral administration (e.g., intramuscular, intravenous, intradermal) will be preferred in special cases, e.g., where oral absorption is impaired as by disease, or the patient is unable to swallow.

The compounds of the present invention are generally administered in the form of pharmaceutical compositions comprising at least one of the compounds of the formula (I), together with a pharmaceutically acceptable vehicle or diluent. Such compositions are generally formulated in a conventional manner utilizing solid or liquid vehicles or diluents as appropriate to the mode of desired administration: for oral administration, in the form of tablets, hard or soft gelatin capsules, suspensions, granules, powders and the like; and, for parenteral administration, in the form of injectable solutions or suspensions, and the like.

The present invention is illustrated by the following examples, but is not limited to the details thereof. Examples 1-3 relate to intermediates for making the compounds of the invention.

EXAMPLE 1

6-(2-Quinolyl)methoxy-4-chromanone

A mixture of 6-hydroxy-4-chromanone (10.0 g, 0.0609 mol), 2-chloromethylquinoline (11.9 g, 0.0670 mol), sodium iodide (10.0 g, 0.0670 mol), potassium carbonate (25.3 g, 0.183 mol), and acetone (200 ml) was refluxed overnight under $N_2$ atmosphere. After 17 hours the reaction appeared lighter and tlc analysis (10% EtOAc/$CH_2Cl_2$) indicated complete conversion of starting material to a slightly less polar product. The mixture was cooled, filtered, and the filtrate concentrated in vacuo. The residue was taken up in ethyl acetate (400 ml), washed with $H_2O$ and brine, dried over $MgSO_4$, and concentrated in vacuo to a dark brown oil. Purification on a silica gel column eluted with 10% ethyl acetate/$CH_2Cl_2$ gave title product as an off-white solid, 15.3 g (82%), m.p. 112-114° C.; tlc (1:9 ethyl acetate:$CH_2Cl_2$) Rf 0.30.

EXAMPLE 2

3-Hydroxymethylene-6-(2-quinolyl)methoxy-4-chromanone

To a solution of title product of the preceding Example (7.00 g, 0.0229 mol) and excess ethyl formate (35 ml) in toluene (80 ml) at room temperature under argon was added in portions over 5 minutes 2.2 g (0.0458 mol) of 50% sodium hydride in mineral oil. The yellow-green mixture was stirred at room temperature for 5 minutes, followed by the addition of 2 drops of ethanol to initiate the reaction. Within 5 minutes the mixture turned red-orange with gas evolution and was mildly exothermic. The mixture was stirred at room temperature for 1 hour, after which tlc (5% $CH_3OH$/$CH_2Cl_2$) indicated complete conversion of starting material to a more polar product. The reaction mixture was poured into 400 ml of ice water, adjusted to pH 5 with 2N HCl, and extracted with ethyl acetate (500 ml). The organic layer was washed with $H_2O$ and brine, dried over $MgSO_4$, and concentrated in vacuo to a pasty yellow solid. Repeated trituration with hexanes to remove mineral oil gave present title product in 85% yield, tlc (1:19 $CH_3OH$:$CH_2Cl_2$) Rf 0.40.

EXAMPLE 3

3-Diazo-6-(2-quinolyl)methoxy-4-chromanone

To a solution of the title product of the preceding Example (7.60 g, 0.023 mol) and dry triethylamine (6.4 ml, 0.046 mol) in dry $CH_2Cl_2$ (100 ml) at -30° C. (dry ice-acetone bath) was added dropwise over 20 minutes a solution of tosyl azide (4.5 g, 0.023 mol) in $CH_2Cl_2$ (25 ml). The reaction mixture was allowed to gradually warm to room temperature overnight with stirring. After 18 hours tlc (20% ethyl acetate/$CH_2Cl_2$) indicated complete disappearance of starting material and formation of a less polar product. The mixture was treated with 1N NaOH (100 ml) and stirred for 10 minutes. After treating with brine, the layers were separated and the organic layer was diluted with 200 ml of ethyl acetate. Methylene chloride was then removed in vacuo. The ethyl acetate residue was washed with $H_2O$ and brine, dried over $MgSO_4$, and concentrated in vacuo to give present title product as a dark yellow solid, 6 g (90%); tlc (1:4 ethyl acetate:$CH_2Cl_2$) Rf 0.27.

EXAMPLE 4

3-Cyclohexyloxy-6-(2-quinolyl)methoxy-4-chromanone

To a suspension of the title product of the preceding Example (1.50 g, 4.53 mmol) and cyclohexanol (1.7 ml, 16.4 mmol) in dry toluene (25 ml) at 70° C. was added 5 mg of rhodium (II) acetate dimer. The reaction quickly evolved $N_2$ and became homogeneous. Tlc analysis (20% ethyl acetate/$CH_2Cl_2$) indicated formation of a less polar product and only a trace of starting material. The reaction mixture was concentrated in vacuo. The residue was taken up in ethyl acetate (100 ml), washed with $H_2O$ and brine, dried over $MgSO_4$, and concentrated in vacuo to an amber oil. Silica gel column chromatography, eluting with 10% ethyl acetate/$CH_2Cl_2$, gave the desired product as a yellow residue, 0.59 g (32%); tlc (1:4 ethyl acetate:$CH_2Cl_2$) Rf 0.68. IR (KBr) 2940, 1700, 1490 $cm^{-1}$. MS (m/e) 403.1780 ($M^+$).

EXAMPLE 5

## cis- and trans-3-Cyclohexyloxy-6-(2-quinolyl)methoxy-4-chromanol

To a solution of the title product of the preceding Example (580 mg, 1.44 mmol) in methanol (30 ml) at 0-5° C. was added 56 mg (1.45 mmol) of sodium borohydride. The reaction mixture was allowed to warm to room temperature with stirring. After 1 hour, tlc (20% ethyl acetate/$CH_2Cl_2$) indicated complete conversion of starting material to two more polar products. The mixture was concentrated in vacuo. The residue was taken up in ethyl acetate, washed with $H_2O$ and brine, dried over $MgSO_4$, and concentrated in vacuo to a yellow-white solid. Silica gel column chromatography eluting with 20% ethyl acetate/$CH_2Cl_2$ afforded less polas cis-title product as a yellow foam (450 mg) and more polar trans-title product as a light yellow oil (30 mg). Total yield = 82%. The cis-isomer was recrystallized from toluene-hexanes to give 417 mg of yellow-white needles, m.p. 127-130° C., and the trans-isomer was triturated with hexanes to give 11 mg of a white solid, m.p. 63-65° C.

cis-isomer. IR (KBr) 1500, 2940 $cm^{-1}$. MS (m/e) 405.1922 ($M^+$).

| Analysis calculated for $C_{25}H_{27}NO_4$: | | | |
|---|---|---|---|
| | C, 74.05; | H, 6.71; | N, 3.45%. |
| Found: | C, 74.07; | H, 6.69; | N, 3.38%. |

trans-isomer. IR (KBr) 1495, 2940 $cm^{-1}$. MS (m/e) 405.1980 ($M^+$).

EXAMPLE 6

3-(1-Methylethoxy)-6-(2-quinolyl)methoxy-4-chromanone

By the methods of Example 4, title product of Example 3 (1.12 g) and isopropyl alcohol were converted to present chromatographed title product, 1.48 g (81%), m.p. 85° C.; tlc (1:9 ethyl acetate:$CH_2Cl_2$) Rf 0.35.

EXAMPLE 7

## cis- and trans-3-(1-Methylethoxy)-6-(2-quinolyl)methoxy-4-chromanol

By the methods of Example 5, title product of the preceding Example (1.38 g) was converted to present chromatographed title products.

cis-isomer. 1.19 g (86%), m.p. 116-118° C., less polar. IR (KBr) 1490 $cm^{-1}$. MS (m/e) 365.1360 ($M^+$).

| Analysis calculated for $C_{22}H_{23}NO_4$: | | | |
|---|---|---|---|
| | C, 72.31; | H, 6.34; | N, 3.83%. |
| Found: | C, 71.95; | H, 6.01; | N, 3.76%. |

trans-isomer. 0.09 g, m.p. 102-103° C., more polar. IR (KBr) 1500 $cm^{-1}$. MS (m/e) 365.1360 ($M^+$).

PREPARATION 1

4-(2-Cyanoethoxy)anisole

4-Methoxyphenol (248 g), KOH (5.6 g) and acrylonitrile (397 ml) were dissolved in 1 liter of t-butanol and heated with stirring at 75° C, for 5 hours. The mixture was then cooled to room temperature and

stripped in vacuo to solid residue, which was repulped in ether and insolubles recovered by filtration. The latter were taken up in 2 liters of ethyl acetate, washed in sequence with 1 liter each of $H_2O$, saturated $NaHCO_3$ and saturated NaCl, dried over $MgSO_4$ and restripped to yield purified title product, 199.4 g, m.p. 62-64° C.

PREPARATION 2

6-Methoxy-4-chromanone

The title product of the preceding Example (199 g) was combined with 240 ml $H_2O$ and 480 ml of concentrated HCl and heated at reflux overnight. The reaction mixture was cooled to room temperature and solids recovered by filtration. The latter were taken up in 2 liters of ethyl acetate, washed with 200 ml of $H_2O$, dried over $MgSO_4$ and stripped in vacuo to yield intermediate 3-(4-methoxyphenoxy)propionic acid, 195 g, m.p. 105-107° C. The latter was added to 600 ml of hot, stirred polyphosphoric acid maintained at 75° C. and the mixture stirred for 2 hours. The temperature rose to a maximum of 89° C. over the first one-half hour, then fell to the 75° C. bath temperature. The reaction mixture was quenched into 3.2 liters of ice and water and extracted with 1.2 liters of ethyl acetate. The organic extract was in sequence with 600 ml each of $H_2O$, saturated $NaHCO_3$ and saturated NaCl, dried over $MgSO_4$ and stripped to 180 g of solids which were taken up in 400 ml $CH_2Cl_2$, treated with activated carbon and restripped to a like quantity of solids. The latter were recrystallized from isopropyl ether to yield purified title product, 120 g, m.p. 46-48° C., identical with the commercial product.

PREPARATION 3

6-Hydroxy-4-chromanone

A solution of 36 g of the product of the preceding Preparation in 290 ml of acetic acid and 290 ml of 48% hydrobromic acid was heated at reflux for 3 hours. The reaction was cooled and stripped in vacuo to crude product which was diluted with water (6 liters), cooled to 0-5° C. and title product recovered by filtration, 25.7 g (80%), m.p. 133-136° C. Optionally, the product is further purified by chromatography on silica gel using ethyl acetate/hexane as eluant.

PREPARATION 4

6-Benzyloxy-4-chromanone

A mixture of 25 g of the product of the preceding Preparation, 26.5 g of benzyl bromide and 28 g of potassium carbonate in 150 ml of acetone was heated at reflux overnight. The reaction was cooled and filtered to remove potassium carbonate. The filtrate was evaporated and the residue was dissolved in ethyl acetate and washed with water. The ethyl acetate layer was dried over sodium sulfate and evaporated in vacuo to obtain the crude product, which was purified by recrystallization from methylene chloride/hexane to give 29 g of title product, m.p. 107-108° C.
$^1$H-NMR(acetone-$d_6$)delta(ppm) : 2.7 (t, 2H), 4.4 (t, 2H), 5.08 (s, 2H), 7.2-7.5 (m, 3H).

PREPARATION 5

3-Hydroxymethylene-6-benzyloxy-4-chromanone

To a solution of 172.5 g of the product of the preceding Preparation in 1.7 liters of toluene containing 168 ml of ethyl formate and 3.5 ml of ethanol was added, in portions, 66 g of 50% sodium hydride. The reaction was allowed to stir at room temperature for 1 hour, then poured into 1.5 liters of ice and $H_2O$, and acidified to pH 4 with dilute hydrochloric acid. The aqueous layer was extracted with several portions of ethyl acetate. The organic layers were combined, dried over sodium sulfate and evaporated in vacuo to give the crude product which was triturated with hexane to remove hydride oil. The resultant product crystallized on standing, m.p. 82-85° C.

PREPARATION 6

3-Diazo-6-benzyloxy-4-chromanone

To a -10° C. solution of 35.3 g of title product of the preceding Preparation in 250 ml of dichloromethane containing 25.2 g of triethylamine was added dropwise a solution of 24.4 g of tosyl azide in 100 ml of dichloromethane. After complete addition, the reaction was allowed to warm to room temperature and stirred overnight. The reaction mixture was washed with water, dried over sodium sulfate and evaporated in vacuo to give the crude product, which was purified by column chromatography on silica gel eluting with dichloromethane to give 21 g of product, m.p. 100-103° C.
$^1$H-NMR($CDCl_3$)delta(ppm): 5.02 (d, J=4, 2H), 6.7-7.5 (m, 10H).

## Claims

**Claims for the following Contracting States : AT, BE, CH, DE, FR, GB, IT, LI, LU, NL, SE**

**1.** A racemic or optically active compound having the structural formula

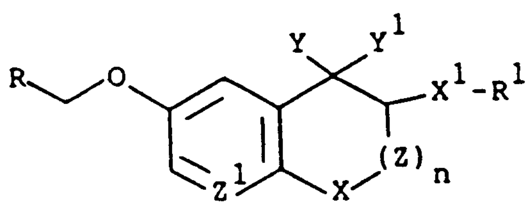

wherein
n is 0 or 1;
X is O, S, SO, $SO_2$, NH or N($C_1$-$C_4$)alkyl;
$X^1$ is $CH_2$, O, S, SO or $SO_2$;
Y and $Y^1$ are taken together and form a carbonyl group, or Y and $Y^1$ are taken separately, Y is hydrogen and $Y^1$ is hydroxy or an acyloxy group which is hydrolyzed to form a hydroxy group under physiological conditions;
Z is $CH_2$, $CHCH_3$, $CH_2CH_2$ or $CH_2CH_2CH_2$;
$Z^1$ is CH or N;
R is 2-, 3- or 4-pyridyl, 2-, 3- or 4-quinolyl, 1-, 3- or 4-isoquinolyl, 3- or 4-pyridazinyl, 3- or 4-cinnolinyl, 1-phthalazinyl, 2- or 4-pyrimidinyl, 2- or 4-quinazolinyl, 2-pyrazinyl, 2-quinoxalinyl, 1-, 2- or 3-indolizinyl, 2-, 4- or 5-oxazolyl, 2-benzoxazolyl, 3-, 4- or 5-isoxazolyl, 5-benzo[c]isoxazolyl, 3-benzo[d]-isoxazolyl, 2-, 4- or 5-thiazolyl, 2-benzothiazolyl, 3-, 4- or 5-isothiazolyl, 5-benzo[c]isothiazolyl, 3-benzo-[d]isothiazolyl, 1-[($C_1$-$C_4$)alkyl]-2-, 4- or 5-imidazolyl, 1-[($C_1$-$C_4$)alkyl]-2-benzimidazolyl, 1-[($C_1$-$C_4$)alkyl]-3-, 4- or 5-pyrazolyl, 2-[($C_1$-$C_4$)alkyl]-3(2H)-indazolyl, or 1-[($C_1$-$C_4$)alkyl]-3(1H)-indazolyl;
or one of said groups mono- or disubstituted on carbon with the same or different substituents which are bromo, chloro, fluoro, ($C_1$-$C_4$)alkyl, trifluoromethyl, hydroxy, hydroxymethyl or ($C_1$-$C_4$)alkoxy, or substituted on adjacent carbons with trimethylene, tetramethylene, -$CH_2$-O-$CH_2$- or -O-$CH_2$-O; and
$R^1$ is ($C_1$-$C_8$)alkyl, ($C_3$-$C_8$)cycloalkyl, ($C_7$-$C_{10}$)bicycloalkyl, ($C_4$-$C_{10}$)cycloalkylalkyl, ($C_8$-$C_{11}$)-bicycloalkylalkyl, or one of said groups mono- or disubstituted with the same or different groups which are fluoro, ($C_1$-$C_4$)alkyl, ($C_1$-$C_4$)alkoxy, carboxy, or [($C_1$-$C_4$)alkoxy]carbonyl;
a pharmaceutically acceptable acid addition salt thereof; or
a pharmaceutically acceptable cationic salt when the compound contains a carboxy group.

**2.** A compound of claim 1 wherein Y and $Y^1$ are taken together and form a carbonyl group.

**3.** A compound of claim 1 wherein Y and $Y^1$ are taken separately, Y is hydrogen, and $Y^1$ is an acyloxy group in which the acyl moiety is the alpha-aminoacyl residue of a naturally occurring L-alpha-amino acid,

$$-\overset{O}{\overset{\|}{C}}-(CH_2)_pNR^2R^3,\quad -\overset{O}{\overset{\|}{C}}-CHNH_2(CH_2)_qNR^2R^3,\quad -\overset{O}{\overset{\|}{C}}-(CH_2)_rCOOH,\ \text{or}$$

$$-\overset{O}{\overset{\|}{C}}-CHNH_2(CH_2)_sCOOH;$$

$R^2$ and $R^3$ are taken separately and are each independently hydrogen or $(C_1$-$C_4)$alkyl, or $R^2$ and $R^3$ are taken together with the nitrogen to which they are attached to form a pyrrolidine, piperidine, perhydroazepin or morpholine ring;
p is an integer from 1 to 4;
q is an integer from 1 to 3;
r is an integer from 2 to 3; and
s is an integer from 1 to 3.

**4.** A compound of claim 1 wherein Y and $Y^1$ are taken separately, Y is hydrogen and $Y^1$ is hydroxy.

**5.** A compound of claim 4 wherein n is 1, Z is $CH_2$, $Z^1$ is CH, X and $X^1$ are each independently $CH_2$ or O, R is 2-pyridyl or 2-quinolyl and $R^1$ is $(C_2$-$C_8)$alkyl or $(C_3$-$C_8)$cycloalkyl.

**6.** A racemic or optically active compound of claim 5 having the relative stereochemical formula

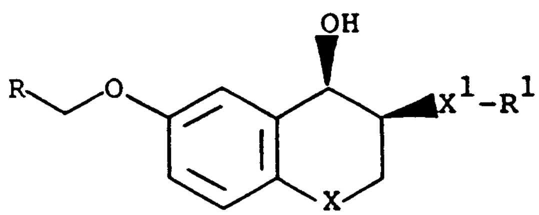 .

**7.** A compound of claim 6 wherein X is O, $X^1$ is O or $CH_2$, R is 2-pyridyl or 2-quinolyl and $R^1$ is propyl, isopropyl or cyclohexyl.

**8.** A racemic or optically active compound of claim 5 having the relative stereochemical formula

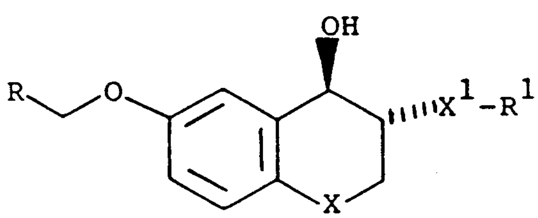 .

**9.** A compound of claim 8 wherein X is O, $X^1$ is O or $CH_2$, R is 2-pyridyl or 2-quinolyl and $R^1$ is propyl, isopropyl or cyclohexyl.

**10.** A compound of the formula

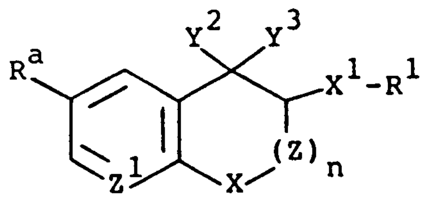

wherein

$R^a$ is hydroxy or benzyloxy;

n is 0 or 1;

X i O, S, SO, $SO_2$, NH or N($C_1$-$C_4$)alkyl;

$X^1$ is $CH_2$, O, S, SO or $SO_2$;

$Z^1$ is CH or N;

$Y^2$ and $Y^3$ are taken together and are carbonyl or $Y^2$ and $Y^3$ are taken separately, $Y^2$ is hydrogen and $Y^3$ is hydroxy; and

$R^1$ is ($C_1$-$C_8$)alkyl, ($C_3$-$C_8$)cycloalkyl, ($C_7$-$C_{10}$)bicycloalkyl, ($C_4$-$C_{10}$)cycloalkylalkyl, ($C_8$-$C_{11}$)-bicycloalkylalkyl, or one of said groups mono- or disubstituted with the same or different groups which are fluoro, ($C_1$-$C_4$)alkyl, ($C_1$-$C_4$)alkoxy, carboxy, or [($C_1$-$C_4$)alkoxy]carbonyl.

**11.** A pharmaceutical composition for administration to a mammal which comprises a 5-lipoxygenase inhibiting and/or leukotriene D4 receptor blocking amount of a compound of claim 1 and a pharmaceutically acceptable carrier.

**12.** A compound according to any one of claims 1 to 9 for inhibiting 5-lipoxygenase enzyme and/or blocking leukotriene receptors.

**13.** Use of a compound according to any one of claims 1 to 9 for making a medicament for inhibiting 5-lipoxygenase enzyme and/or blocking leukotriene receptors.

**Claims for the following Contracting State : ES**

**1.** A process for a racemic or optically active compound having the structural formula

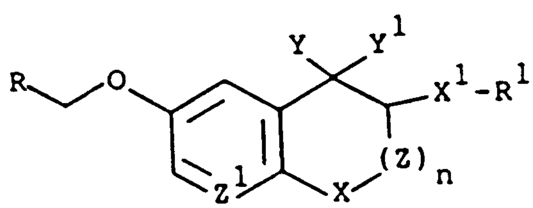

wherein

n is 0 or 1;

X is O, S, SO, $SO_2$, NH or N($C_1$-$C_4$)alkyl;

$X^1$ is $CH_2$, O, S, SO or $SO_2$;

Y and $Y^1$ are taken together and form a carbonyl group, or Y and $Y^1$ are taken separately, Y is hydrogen and $Y^1$ is hydroxy or an acyloxy group which is hydrolyzed to form a hydroxy group under physiological conditions;

Z is $CH_2$, $CHCH_3$, $CH_2CH_2$ or $CH_2CH_2CH_2$;

$Z^1$ is CH or N;

R is 2-, 3- or 4-pyridyl, 2-, 3- or 4-quinolyl, 1-, 3- or 4-isoquinolyl, 3- or 4-pyridazinyl, 3- or 4-cinnolinyl, 1-phthalazinyl, 2- or 4-pyrimidinyl, 2- or 4-quinazolinyl, 2-pyrazinyl, 2-quinoxalinyl, 1-, 2- or 3-indolizinyl, 2-, 4- or 5-oxazolyl, 2-benzoxazolyl, 3-, 4- or 5-isoxazolyl, 5-benzo[c]isoxazolyl, 3-benzo[d]-

isoxazolyl, 2-, 4- or 5-thiazolyl, 2-benzothiazolyl, 3-, 4- or 5-isothiazolyl, 5-benzo[c]isothiazolyl, 3-benzo-[d]isothiazolyl, 1-[($C_1$-$C_4$)alkyl]-2-, 4- or 5-imidazolyl, 1-[($C_1$-$C_4$)alkyl]-2-benzimidazolyl, 1-[($C_1$-$C_4$)alkyl]-3-, 4- or 5-pyrazolyl, 2-[($C_1$-$C_4$)alkyl]-3(2H)-indazolyl, or 1-[($C_1$-$C_4$)alkyl]-3(1H)-indazolyl;
or one of said groups mono- or disubstituted on carbon with the same or different substituents which are bromo, chloro, fluoro, ($C_1$-$C_4$)alkyl, trifluoromethyl, hydroxy, hydroxymethyl or ($C_1$-$C_4$)alkoxy, or substituted on adjacent carbons with trimethylene, tetramethylene, $-CH_2-O-CH_2-$ or $-O-CH_2-O$; and

$R^1$ is ($C_1$-$C_8$)alkyl, ($C_3$-$C_8$)cycloalkyl, ($C_7$-$C_{10}$)bicycloalkyl, ($C_4$-$C_{10}$)cycloalkylalkyl, ($C_8$-$C_{11}$)-bicycloalkylalkyl, or one of said groups mono- or disubstituted with the same or different groups which are fluoro, ($C_1$-$C_4$)alkyl, ($C_1$-$C_4$)alkoxy, carboxy, or [($C_1$-$C_4$)alkoxy]carbonyl;

a pharmaceutically acceptable acid addition salt thereof; or

a pharmaceutically acceptable cationic salt when the compound contains a carboxy group; which comprises:

(a) reaction of a compound of the formula

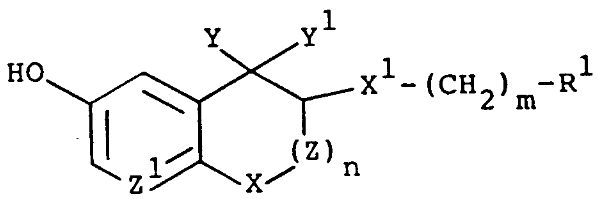

with a compound of the formula

$R-CH_2-X^2$,

where $X^2$ is a nucleophilically displaceable group, in the presence of a base;

(b) when Y and $Y^1$ are taken separately, Y is hydrogen and $Y^1$ is hydroxy, reduction of a preformed compound of the formula (I) wherein Y and $Y^1$ are taken together and form a carbonyl group;

(c) when Y and $Y^1$ are taken together and form a carbonyl group and $X^1$ is O or S,

(i) reaction of a compound of the formula

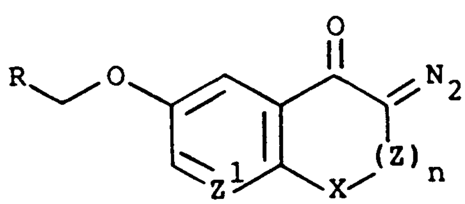

with a compound of the formula

$R^1(CH_2)_mSH$ or $R^1(CH_2)_mOH$

in the presence of rhodium (II) acetate dimer; or

(ii) reaction of a compound of the formula

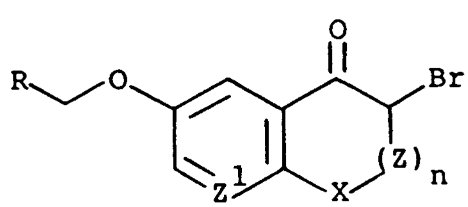

with a compound of the formula

$R^1(CH_2)_mSH$ or $R^1(CH_2)_mOH$

in the presence of a base;

(d) when Y and $Y^1$ are taken together and form a carbonyl group and $X^1$ is $CH_2$, hydrogenation of a compound of the formula

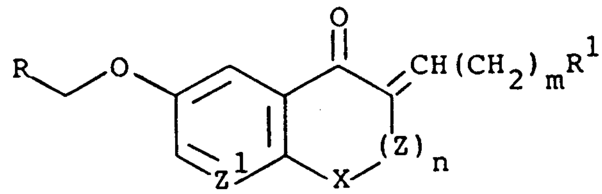

;

or

(e) when $X^1$ is SO or $SO_2$, oxidation of a preformed compound of the formula (I) wherein $X^1$ is S;

(f) when Y and $Y^1$ are taken separately, Y is hydrogen and $Y^1$ is an acyloxy group, acylation of a preformed compound of the formula (I) wherein Y and $Y^1$ are taken separately, Y is hydrogen and $Y^1$ is hydroxy; and, if desired,

(g) converting a preformed compound of the formula (I) to a pharmaceutically-acceptable acid addition salt, or, when it contains a carboxy group, to a pharmaceutically-acceptable cationic salt.

**2.** A process of claim 1 for a compound wherein Y and $Y^1$ are taken together and form a carbonyl group.

**3.** A process of claim 1 for a compound wherein Y and $Y^1$ are taken separately, Y is hydrogen and $Y^1$ is an acyloxy group in which the acyl moiety is the alpha-aminoacyl residue of a naturally occurring L-alpha-amino acid,

$$-\overset{O}{\overset{\|}{C}}-(CH_2)_pNR^2R^3,\quad -\overset{O}{\overset{\|}{C}}-CHNH_2(CH_2)_qNR^2R^3,\quad -\overset{O}{\overset{\|}{C}}-(CH_2)_rCOOH,\ \text{or}$$

$$-\overset{O}{\overset{\|}{C}}-CHNH_2(CH_2)_sCOOH;$$

$R^2$ and $R^3$ are taken separately and are each independently hydrogen or $(C_1\text{-}C_4)$alkyl, or $R^2$ and $R^3$ are taken together with the nitrogen to which they are attached to form a pyrrolidine, piperidine, perhydroazepin or morpholine ring;

p is an integer from 1 to 4;

q is an integer from 1 to 3;

r is an integer from 2 to 3; and

s is an integer from 1 to 3.

**4.** A process of claim 1 for a compound wherein Y and $Y^1$ are taken separately, Y is hydrogen and $Y^1$ is hydroxy.

**5.** A process of claim 4 wherein n is 1, Z is $CH_2$, $Z^1$ is CH, X is O, $X^1$ is $CH_2$ or O, R is 2-pyridyl or 2-quinolyl and $R^1$ is $(C_2\text{-}C_8)$alkyl or $(C_3\text{-}C_8)$cycloalkyl.

**6.** A process of claim 5 for a racemic or optically active compound having the relative stereochemical formula

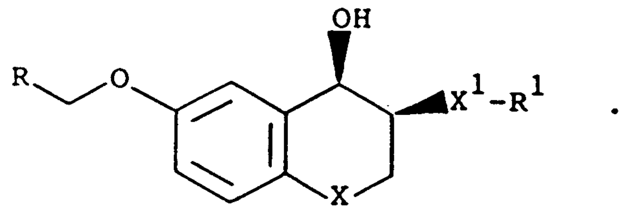

7. A process of claim 6 for a compound wherein X is O, $X^1$ is O or $CH_2$, R is 2-pyridyl or 2-quinolyl and $R^1$ is propyl, isopropyl or cyclohexyl.

8. A process of claim 5 for a racemic or optically active compound having the relative stereochemical formula

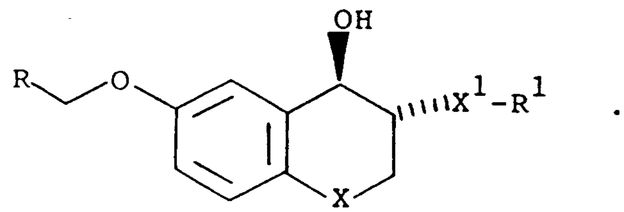

9. A process of claim 8 for a compound wherein X is O, $X^1$ is O or $CH_2$, R is 2-pyridyl or 2-quinolyl and $R^1$ is propyl, isopropyl or cyclohexyl.

**Claims for the following Contracting State : GR**

1. A process for a racemic or optically active compound having the structural formula

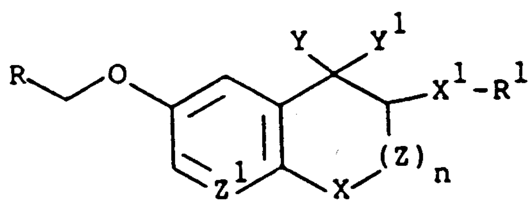

wherein

n is 0 or 1;

X is O, S, SO, $SO_2$, NH or N($C_1$-$C_4$)alkyl;

$X^1$ is $CH_2$, O, S, SO or $SO_2$;

Y and $Y^1$ are taken together and form a carbonyl group, or Y and $Y^1$ are taken separately, Y is hydrogen and $Y^1$ is hydroxy or an acyloxy group which is hydrolyzed to form a hydroxy group under physiological conditions;

Z is $CH_2$, $CHCH_3$, $CH_2CH_2$ or $CH_2CH_2CH_2$;

$Z^1$ is CH or N;

R is 2-, 3- or 4-pyridyl, 2-, 3- or 4-quinolyl, 1-, 3- or 4-isoquinolyl, 3- or 4-pyridazinyl, 3- or 4-cinnolinyl, 1-phthalazinyl, 2- or 4-pyrimidinyl, 2- or 4-quinazolinyl, 2-pyrazinyl, 2-quinoxalinyl, 1-, 2- or 3-indolizinyl, 2-, 4- or 5-oxazolyl, 2-benzoxazolyl, 3-, 4- or 5-isoxazolyl, 5-benzo[c]isoxazolyl, 3-benzo[d]-isoxazolyl, 2-, 4- or 5-thiazolyl, 2-benzothiazolyl, 3-, 4- or 5-isothiazolyl, 5-benzo[c]isothiazolyl, 3-benzo-[d]isothiazolyl, 1-[($C_1$-$C_4$)alkyl]-2-, 4- or 5-imidazolyl, 1-[($C_1$-$C_4$)alkyl]-2-benzimidazolyl, 1-[($C_1$-$C_4$)alkyl]-3-, 4- or 5-pyrazolyl, 2-[($C_1$-$C_4$)alkyl]-3(2H)-indazolyl, or 1-[($C_1$-$C_4$)alkyl]-3(1H)-indazolyl;

or one of said groups mono- or disubstituted on carbon with the same or different substituents which are bromo, chloro, fluoro, $(C_1-C_4)$alkyl, trifluoromethyl, hydroxy, hydroxymethyl or $(C_1-C_4)$alkoxy, or substituted on adjacent carbons with trimethylene, tetramethylene, $-CH_2-O-CH_2-$ or $-O-CH_2-O$; and

$R^1$ is $(C_1-C_8)$alkyl, $(C_3-C_8)$cycloalkyl, $(C_7-C_{10})$bicycloalkyl, $(C_4-C_{10})$cycloalkylalkyl, $(C_8-C_{11})$-bicycloalkylalkyl, or one of said groups mono- or disubstituted with the same or different groups which are fluoro, $(C_1-C_4)$alkyl, $(C_1-C_4)$alkoxy, carboxy, or $[(C_1-C_4)$alkoxy]carbonyl;

a pharmaceutically acceptable acid addition salt thereof; or

a pharmaceutically acceptable cationic salt when the compound contains a carboxy group; which comprises:

(a) reaction of a compound of the formula

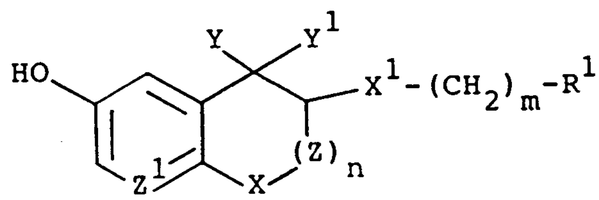

with a compound of the formula

$R-CH_2-X^2$,

where $X^2$ is a nucleophilically displaceable group, in the presence of a base;

(b) when Y and $Y^1$ are taken separately, Y is hydrogen and $Y^1$ is hydroxy, reduction of a preformed compound of the formula (I) wherein Y and $Y^1$ are taken together and form a carbonyl group;

(c) when Y and $Y^1$ are taken together and form a carbonyl group and $X^1$ is O or S,

(i) reaction of a compound of the formula

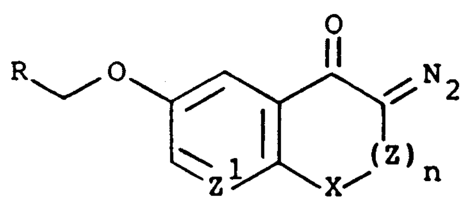

with a compound of the formula

$R^1(CH_2)_mSH$ or $R^1(CH_2)_mOH$

in the presence of rhodium (II) acetate dimer; or

(ii) reaction of a compound of the formula

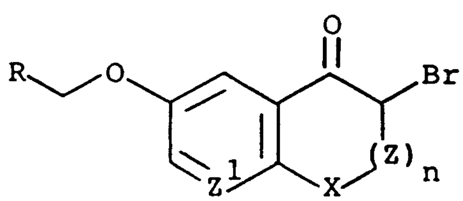

with a compound of the formula

$R^1(CH_2)_mSH$ or $R^1(CH_2)_mOH$

in the presence of a base;

(d) when Y and $Y^1$ are taken together and form a carbonyl group and $X^1$ is $CH_2$, hydrogenation of a compound of the formula

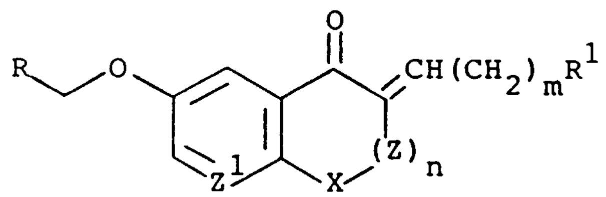

;

or

(e) when $X^1$ is SO or $SO_2$, oxidation of a preformed compound of the formula (I) wherein $X^1$ is S;

(f) when Y and $Y^1$ are taken separately, Y is hydrogen and $Y^1$ is an acyloxy group, acylation of a preformed compound of the formula (I) wherein Y and $Y^1$ are taken separately, Y is hydrogen and $Y^1$ is hydroxy; and, if desired,

(g) converting a preformed compound of the formula (I) to a pharmaceutically-acceptable acid addition salt, or, when it contains a carboxy group, to a pharmaceutically-acceptable cationic salt.

**2.** A process of claim 1 for a compound wherein Y and $Y^1$ are taken together and form a carbonyl group.

**3.** A process of claim 1 for a compound wherein Y and $Y^1$ are taken separately, Y is hydrogen and $Y^1$ is an acyloxy group in which the acyl moiety is the alpha-aminoacyl residue of a naturally occurring L-alpha-amino acid,

$$-\overset{O}{\overset{\|}{C}}-(CH_2)_pNR^2R^3, \quad -\overset{O}{\overset{\|}{C}}-CHNH_2(CH_2)_qNR^2R^3, \quad -\overset{O}{\overset{\|}{C}}-(CH_2)_rCOOH, \text{ or}$$

$$-\overset{O}{\overset{\|}{C}}-CHNH_2(CH_2)_sCOOH;$$

$R^2$ and $R^3$ are taken separately and are each independently hydrogen or $(C_1\text{-}C_4)$alkyl, or $R^2$ and $R^3$ are taken together with the nitrogen to which they are attached to form a pyrrolidine, piperidine, perhydroazepin or morpholine ring;

p is an integer from 1 to 4;

q is an integer from 1 to 3;

r is an integer from 2 to 3; and

s is an integer from 1 to 3.

**4.** A process of claim 1 for a compound wherein Y and $Y^1$ are taken separately, Y is hydrogen and $Y^1$ is hydroxy.

**5.** A process of claim 4 wherein n is 1, Z is $CH_2$, $Z^1$ is CH, X is O, $X^1$ is $CH_2$ or O, R is 2-pyridyl or 2-quinolyl and $R^1$ is $(C_2\text{-}C_8)$alkyl or $(C_3\text{-}C_8)$cycloalkyl.

**6.** A process of claim 5 for a racemic or optically active compound having the relative stereochemical formula

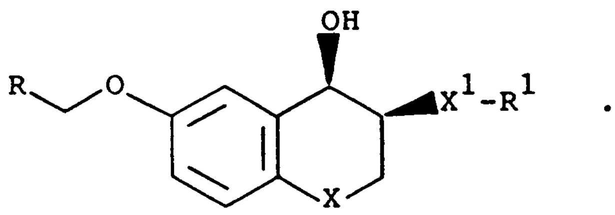

7. A process of claim 6 for a compound wherein X is O, $X^1$ is O or $CH_2$, R is 2-pyridyl or 2-quinolyl and $R^1$ is propyl, isopropyl or cyclohexyl.

8. A process of claim 5 for a racemic or optically active compound having the relative stereochemical formula

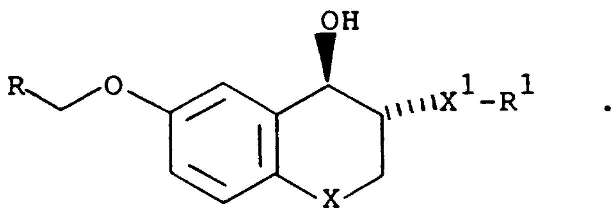

9. A process of claim 8 for a compound wherein X is O, $X^1$ is O or $CH_2$, R is 2-pyridyl or 2-quinolyl and $R^1$ is propyl, isopropyl or cyclohexyl.

10. A compound of the formula

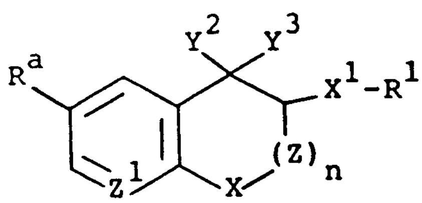

wherein

$R^a$ is hydroxy or benzyloxy;

n is 0 or 1;

X is O, S, SO, $SO_2$, NH or $N(C_1\text{-}C_4)$alkyl;

$X^1$ is $CH_2$, O, S, SO or $SO_2$;

$Z^1$ is CH or N;

$Y^2$ and $Y^3$ are taken together and are carbonyl or $Y^2$ and $Y^3$ are taken separately, $Y^2$ is hydrogen and $Y^3$ is hydroxy; and

$R^1$ is $(C_1\text{-}C_8)$alkyl, $(C_3\text{-}C_8)$cycloalkyl, $(C_7\text{-}C_{10})$bicycloalkyl, $(C_4\text{-}C_{10})$cycloalkylalkyl, $(C_8\text{-}C_{11})$-bicycloalkylalkyl, or one of said groups mono- or disubstituted with the same or different groups which are fluoro, $(C_1\text{-}C_4)$alkyl, $(C_1\text{-}C_4)$alkoxy, carboxy, or $[(C_1\text{-}C_4)$alkoxy]carbonyl.

**Patentansprüche**
**Patentansprüche für folgende Vertragsstaaten : AT, BE, CH, DE, FR, GB, IT, LI, LU, NL, SE**

1. Racemische oder optisch aktive Verbindung mit der Strukturformel

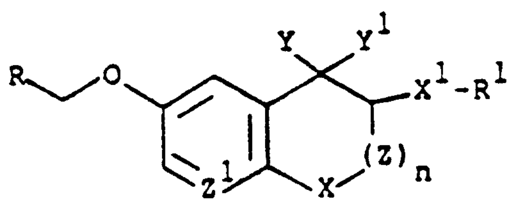

worin
n Null oder eins ist;
X O, S, SO, $SO_2$, NO oder N($C_1$-$C_4$)Alkyl ist;
$X^1$ $CH_2$, O, S, SO oder $SO_2$ ist;
Y und $Y^1$ zusammen genommen werden und eine Carbonylgruppe bilden; oder Y und $Y^1$ werden getrennt genommen, und Y ist Wasserstoff, und $Y^1$ ist Hydroxy oder eine Acyloxygruppe, die unter physiologischen Bedingungen hydrolysiert wird, um eine Hydroxygruppe zu bilden;
Z $CH_2$, $CHCH_3$, $CH_2CH_2$ oder $CH_2CH_2CH_2$ ist;
$Z^1$ CH oder N ist;
R 2-, 3- oder 4-Pyridyl, 2-, 3- oder 4-Chinolyl, 1-, 3- oder 4-Isochinolyl, 3- oder 4-Pyridazinyl, 3- oder 4-Cinnolinyl, 1-Phthalazinyl, 2- oder 4-Pyrimidinyl, 2- oder 4-Chinazolinyl, 2-Pyrazinyl, 2-Chinoxalinyl, 1-, 2- oder 3-Indolizinyl, 2-, 4- oder 5-Oxazolyl, 2-Benzoxazolyl, 3-, 4- oder 5-Isoxazolyl, 5-Benzo[c]-isoxazolyl, 3-Benzo[d]isoxazolyl, 2-, 4- oder 5-Thiazolyl, 2-Benzothiazolyl, 3-, 4- oder 5-Isothiazolyl, 5-Benzo[c]isothiazolyl, 3-Benzo[d]isothiazolyl, 1-[($C_1$-$C_4$)Alkyl]-2-, -4- oder -5-imidazolyl, 1-[($C_1$-$C_4$)Alkyl]-2-benzimidazolyl, 1-[($C_1$-$C_4$)Alkyl]-3-, -4- oder -5-pyrazolyl, 2-[($C_1$-$C_4$)Alkyl]-3-(2H)-indazolyl oder 1-[-($C_1$-$C_4$)Alkyl]-3(1H)-indazolyl ist; oder eine dieser Gruppen ist am Kohlenstoff mit dem gleichen oder unterschiedlichen Substituenten, die Brom, Chlor, Fluor, ($C_1$-$C_4$)Alkyl, Trifluormethyl, Hydroxy, Hydroxymethyl oder ($C_1$-$C_4$)Alkoxy sind, mono- oder disubstituiert oder an benachbarten Kohlenstoffen mit Trimethylen, Tetramethylen, -$CH_2$-0-$CH_2$- oder -O-$CH_2$-0 substituiert; und
$R^1$ ($C_1$-$C_8$)Alkyl, ($C_3$-$C_8$)Cycloalkyl, ($C_7$-$C_{10}$)Bicycloalkyl, ($C_4$-$C_{10}$)Cycloalkylalkyl, ($C_8$-$C_{11}$)-Bicycloalkylalkyl ist, oder eine dieser Gruppen ist mit der gleichen oder unterschiedlichen Gruppen, die Fluor, ($C_1$-$C_4$)Alkyl, ($C_1$-$C_4$)Alkoxy, Carboxy oder [($C_1$-$C_4$)Alkoxy]-carbonyl sind, mono- oder disubstituiert;
oder ein pharmazeutisch annehmbares Säureadditionssalz davon oder
oder ein pharmazeutisch annehmbares Kationensalz, wenn die Verbindung eine Carboxygruppe enthält.

2. Verbindung nach Anspruch 1, worin Y und $Y^1$ zusammen genommen werden und eine Carbonylgruppe bilden.

3. Verbindung nach Anspruch 1, worin Y und $Y^1$ genommen werden, Y Wasserstoff ist und $Y^1$ eine Acyloxygruppe ist, in welcher der Acylteil der α-Aminoacylrest einer natürlich vorkommenden L-α-Aminosäure

$$-\overset{O}{\overset{\|}{C}}-(CH_2)_pNR^2R^3,\quad -\overset{O}{\overset{\|}{C}}-CHNH_2(CH_2)_qNR^2R^3,\quad -\overset{O}{\overset{\|}{C}}-(CH_2)_rCOOH \text{ oder}$$

$$-\overset{O}{\overset{\|}{C}}-CHNH_2(CH_2)_sCOOH$$

ist;
$R^2$ und $R^3$ getrennt genommen werden und jeweils unabhängig Wasserstoff oder ($C_1$-$C_4$)Alkyl sind, oder $R^2$ und $R^3$ zusammen mit dem Stickstoffatom, an welches sie gebunden sind, genommen werden und einen Pyrrolidin-, Piperidin-, Perhydroazepin- oder Morpholinring bilden;
p eine ganze Zahl von 1 bis 4 ist;
q eine ganze Zahl von 1 bis 3 ist;
r eine ganze Zahl von 2 bis 3 ist; und
s eine ganze Zahl von 1 bis 3 ist.

**4.** Verbindung nach Anspruch 1, worin Y und $Y^1$ getrennt genommen werden und Y Wasserstoff und $Y^1$ Hydroxy ist.

**5.** Verbindung nach Anspruch 1, worin n 1 ist, Z $CH_2$ ist, $Z^1$ CH ist, X und $X^1$ jeweils unabhängig $CH_2$ oder O sind, R 2-Pyridyl oder 2-Chinolyl ist und $R^1$ ($C_2$-$C_8$)Alkyl oder ($C_3$-$C_8$)Cycloalkyl ist.

**6.** Racemische oder optisch aktive Verbindung nach Anspruch 5 mit der relativen stereochemischen Formel

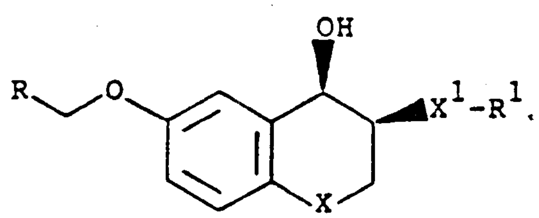

**7.** Verbindung nach Anspruch 6, worin X O ist, $X^1$ O oder $CH_2$ ist, R 2-Pyridyl oder 2-Chinoyl ist und $R^1$ Propyl, Isopropyl oder Cyclohexyl ist.

**8.** Racemische oder optisch aktive Verbindung nach Anspruch 5 mit der relativen stereochemischen Formel

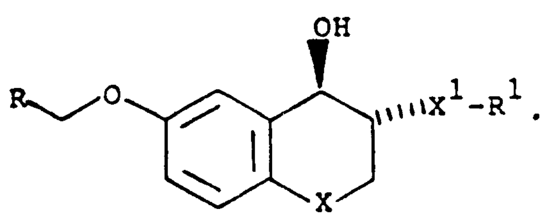

**9.** Verbindung nach Anspruch 8, worin X O ist, $X^1$ O oder $CH_2$ ist, R 2-Pyridyl oder 2-Chinolyl ist und $R^1$ Propyl, Isopropyl oder Cyclohexyl ist.

**10.** Verbindung mit der Formel

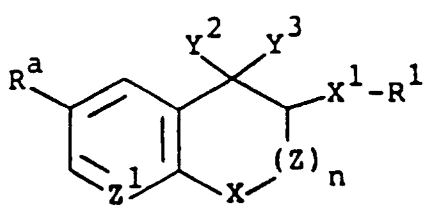

worin

$R^a$ Hydroxy oder Benzyloxy ist,

n = 0 oder 1 ist,

X O, S, SO, $SO_2$, NH oder $N(C_1-C_4)$Alkyl ist,

$X^1$ $CH_2$, O, S, SO oder $SO_2$ ist,

$Z^1$ CH oder N ist,

$Y^2$ und $Y^3$ zusammen genommen werden und Carbonyl sind oder $Y^2$ und $Y^3$ getrennt genommen werden und $Y^2$ Wasserstoff und $Y^3$ Hydroxy ist und

$R^1$ $(C_1-C_8)$Alkyl, $(C_3-C_8)$Cycloalkyl, $(C_7-C_{10})$Bicycloalkyl, $(C_4-C_{10})$Cycloalkylalkyl, $(C_8-C_{11})$-Bicycloalkylalkyl oder eine dieser Gruppen ist, die mit der gleichen oder unterschiedlichen Gruppen, die Fluor, $(C_1-C_4)$Alkyl, $(C_1-C_4)$Alkoxy, Carboxy oder $[(C_1-C_4)$Alkoxy]carbonyl sind, mono- oder disubstituiert ist.

**11.** Pharmazeutische Zusammensetzung zur Verabreichung an einen Säuger, die eine 5-Lipoxygenase inhibierende und/oder Leukotrien D4-Rezeptoren blockierende Menge einer Verbindung nach Anspruch 1 und einen pharmazeutisch annehmbaren Träger umfaßt.

**12.** Verbindung nach irgendeinem der Ansprüche 1 bis 9 zum Inhibieren von 5-Lipoxygenase-enzym und/oder zum Blockieren von Leukotrienrezeptoren.

**13.** Verwendung einer Verbindung nach irgendeinem der Ansprüche 1 bis 9 zur Herstellung eines Arzneimittels zum Inhibieren von 5-Lipoxygenase-enzym und/oder zum Blockieren von Leukotrienrezeptoren.

**Patentansprüche für folgenden Vertragsstaat : ES**

**1.** Verfahren zur Herstellung einer racemischen oder optisch aktiven Verbindung mit der Strukturformel

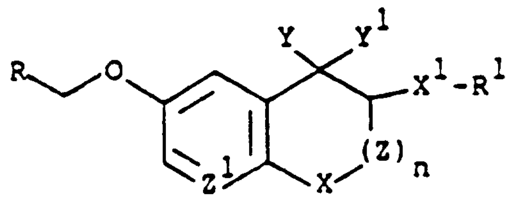

worin

n Null oder eins ist;

X O, S, SO, $SO_2$, NO oder $N(C_1-C_4)$Alkyl ist;

$X^1$ $CH_2$, O, S, SO oder $SO_2$ ist;

Y und $Y^1$ zusammen genommen werden und eine Carbonylgruppe bilden; oder Y und $Y^1$ werden getrennt genommen, und Y ist Wasserstoff, und $Y^1$ ist Hydroxy oder eine Acyloxygruppe, die unter physiologischen Bedingungen hydrolysiert wird, um eine Hydroxygruppe zu bilden;

Z $CH_2$, $CHCH_3$, $CH_2CH_2$ oder $CH_2CH_2CH_2$ ist;

$Z^1$ CH oder N ist;

R 2-, 3- oder 4-Pyridyl, 2-, 3- oder 4-Chinolyl, 1-, 3- oder 4-Isochinolyl, 3- oder 4-Pyridazinyl, 3- oder 4-Cinnolinyl, 1-Phthalazinyl, 2- oder 4-Pyrimidinyl, 2- oder 4-Chinazolinyl, 2-Pyrazinyl, 2-Chinoxalinyl, 1-, 2- oder 3-Indolizinyl, 2-, 4- oder 5-Oxazolyl, 2-Benzoxazolyl, 3-, 4- oder 5-Isoxazolyl, 5-Benzo[c]-isoxazolyl, 3-Benzo[d]isoxazolyl, 2-, 4- oder 5-Thiazolyl, 2-Benzothiazolyl, 3-, 4- oder 5-Isothiazolyl, 5-Benzo[c]isothiazolyl, 3-Benzo[d]isothiazolyl, 1-[$(C_1-C_4)$Alkyl]-2-, -4- oder -5-imidazolyl, 1-[$(C_1-C_4)$Alkyl]-2-benzimidazolyl, 1-[$(C_1-C_4)$Alkyl]-3-, -4- oder -5-pyrazolyl, 2-[$(C_1-C_4)$Alkyl]-3(2H)-indazolyl oder 1-[$(C_1-C_4)$Alkyl]-3(1H)indazolyl ist; oder eine dieser Gruppen ist am Kohlenstoff mit dem gleichen oder unterschiedlichen Substituenten, die Brom, Chlor, Fluor, $(C_1-C_4)$Alkyl, Trifluormethyl, Hydroxy, Hydroxymethyl oder $(C_1-C_4)$Alkoxy sind, mono- oder disubstituiert oder an benachbarten Kohlenstoffen mit Trimethylen, Tetramethylen, $-CH_2-0-CH_2-$ oder $-O-CH_2-0$ substituiert; und

$R^1$ $(C_1-C_8)$Alkyl, $(C_3-C_8)$Cycloalkyl, $(C_7-C_{10})$Bicycloalkyl, $(C_4-C_{10})$Cycloalkylalkyl, $(C_8-C_{11})$-Bicycloalkylalkyl ist, oder eine dieser Gruppen ist mit der gleichen oder unterschiedlichen Gruppen, die

Fluor, $(C_1-C_4)$Alkyl, $(C_1-C_4)$Alkoxy, Carboxy oder $[(C_1-C_4)$Alkoxy]-carbonyl sind, mono- oder disubstituiert;
oder eines pharmazeutisch annehmbaren Säureadditionssalzes derselben oder
eines pharmazeutisch annehmbaren Kationensalzes, wenn die Verbindung eine Carboxygruppe enthält, das umfaßt:

(a) Umsetzung einer Verbindung der Formel

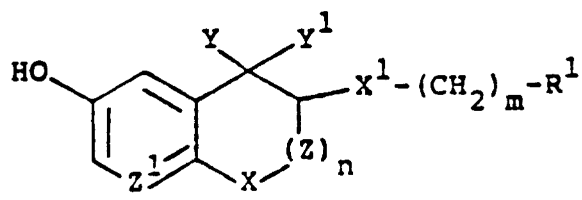

mit einer Verbindung der Formel

$R-CH_2-X^2$

worin $X^2$ eine nukleophil ersetzbare Gruppe ist, in Gegenwart einer Base;

(b) wenn Y und $Y^1$ getrennt genommen werden und Y Wasserstoff und $Y^1$ Hydroxy ist, Reduktion einer vorgebildeten Verbindung der Formel (I), worin Y und $Y^1$ zusammen genommen werden und eine Carbonylgruppe bilden;

(c) wenn Y und $Y^1$ zusammen genommen werden und eine Carbonylgruppe bilden und $X^1$ O oder S ist,

(i) Umsetzung einer Verbindung der Formel

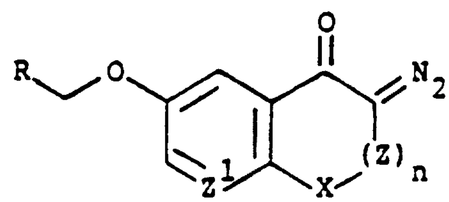

mit einer Verbindung der Formel

$R^1(CH_2)_mSH$ oder $R^1(CH_2)_mOH$

in Gegenwart von Rhodium(II)acetat-dimer; oder

(ii) Umsetzung einer Verbindung der Formel

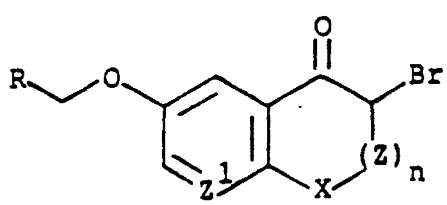

mit einer Verbindung der Formel

$R^1(CH_2)_mSH$ oder $R^1(CH_2)_mOH$

in Gegenwart einer Base;

(d) wenn Y und $Y^1$ zusammen genommen werden und eine Carbonylgruppe bilden und $X^1$ $CH_2$ ist, Hydrierung einer Verbindung der Formel

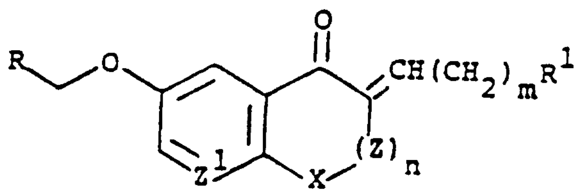

oder

(e) wenn $X^1$ SO oder $SO_2$ ist, Oxidation einer vorgebildeten Verbindung der Formel (I), worin $X^1$ S ist;

(f) wenn Y und $Y^1$ getrennt genommen werden und Y Wasserstoff und $Y^1$ eine Acyloxygruppe ist, Acylierung einer vorgebildeten Verbindung der Formel (I), worin Y und $Y^1$ getrennt genommen werden und Y Wasserstoff und $Y^1$ Hydroxy ist, und, falls gewünscht,

(g) Umwandeln einer vorgebildeten Verbindung der Formel (I) in ein pharmazeutisch annehmbares Säureadditionssalz oder, wenn sie eine Carboxygruppe enthält, in ein pharmazeutisch annehmbares Kationensalz.

**2.** Verfahren nach Anspruch 1 für eine Verbindung, in welcher Y und $Y^1$ zusammen genommen werden und eine Carbonylgruppe bilden.

**3.** Verfahren nach Anspruch 1 für eine Verbindung, worin Y und $Y^1$ getrennt genommen werden und Y Wasserstoff und $Y^1$ eine Acyloxygruppe ist, in welcher der Acylteil der α-Aminoacylrest einer natürlich vorkommenden L-α-Aminosäure,

$$-\overset{O}{\overset{\|}{C}}-(CH_2)_pNR^2R^3,\quad -\overset{O}{\overset{\|}{C}}-CHNH_2(CH_2)_qNR^2R^3,\quad -\overset{O}{\overset{\|}{C}}-(CH_2)_rCOOH \text{ oder}$$

$$-\overset{O}{\overset{\|}{C}}-CHNH_2(CH_2)_sCOOH$$

ist;

$R^2$ und $R^3$ getrennt genommen werden und jeweils unabhängig Wasserstoff oder $(C_1\text{-}C_4)$Alkyl sind, oder $R^2$ und $R^3$ zusammen mit dem Stickstoffatom, an welches sie gebunden sind, genommen werden und einen Pyrrolidin-, Piperidin-, Perhydroazepin- oder Morpholinring bilden;

p eine ganze Zahl von 1 bis 4 ist;

q eine ganze Zahl von 1 bis 3 ist;

r eine ganze Zahl von 2 bis 3 ist; und

s eine ganze Zahl von 1 bis 3 ist.

**4.** Verfahren nach Anspruch 1 für eine Verbindung, worin Y und $Y^1$ getrennt genommen werden und Y Wasserstoff und $Y^1$ Hydroxy ist.

**5.** Verfahren nach Anspruch 1, worin n 1 ist, Z $CH_2$ ist, $Z^1$ CH ist, X O ist, $X^1$ $CH_2$ oder O ist, R 2-Pyridyl oder 2-Chinolyl ist und $R^1$ $(C_2\text{-}C_8)$Alkyl oder $(C_3\text{-}C_8)$-Cycloalkyl ist.

**6.** Verfahren nach Anspruch 5 für eine racemische oder optisch aktive Verbindung mit der relativen stereochemischen Formel

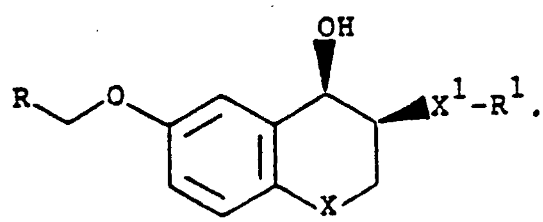

7. Verfahren nach Anspruch 6 für eine Verbindung, worin X O ist, $X^1$ O oder $CH_2$ ist, R 2-Pyridyl oder 2-Chinoyl ist und $R^1$ Propyl, Isopropyl oder Cyclohexyl ist.

8. Verfahren nach Anspruch 5 für eine racemische oder optisch aktive Verbindung mit der relativen stereochemischen Formel

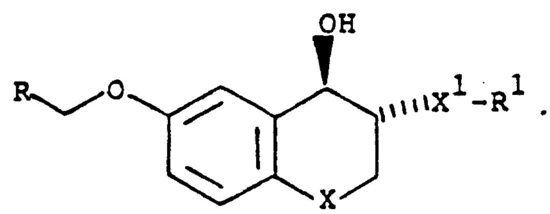

9. Verfahren nach Anspruch 8 für eine Verbindung, worin X O ist, $X^1$ O oder $CH_2$ ist, R 2-Pyridyl oder 2-Chinolyl ist und $R^1$ Propyl, Isopropyl oder Cyclohexyl ist.

**Patentansprüche für folgenden Vertragsstaat : GR**

1. Verfahren zur Herstellung einer racemischen oder optisch aktiven Verbindung mit der Strukturformel

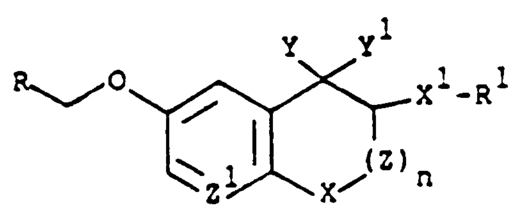

worin
n Null oder eins ist;
X O, S, SO, $SO_2$, NO oder $N(C_1$-$C_4)$Alkyl ist;
$X^1$ $CH_2$, O, S, SO oder $SO_2$ ist;
Y und $Y^1$ zusammen genommen werden und eine Carbonylgruppe bilden; oder Y und $Y^1$ werden getrennt genommen, und Y ist Wasserstoff, und $Y^1$ ist Hydroxy oder eine Acyloxygruppe, die unter physiologischen Bedingungen hydrolysiert wird, um eine Hydroxygruppe zu bilden;
Z $CH_2$, $CHCH_3$, $CH_2CH_2$ oder $CH_2CH_2CH_2$ ist;
$Z^1$ CH oder N ist;
R 2-, 3- oder 4-Pyridyl, 2-, 3- oder 4-Chinolyl, 1-, 3- oder 4-Isochinolyl, 3- oder 4-Pyridazinyl, 3- oder 4-Cinnolinyl, 1-Phthalazinyl, 2- oder 4-Pyrimidinyl, 2- oder 4-Chinazolinyl, 2-Pyrazinyl, 2-Chinoxalinyl, 1-, 2- oder 3-Indolizinyl, 2-, 4- oder 5-Oxazolyl, 2-Benzoxazolyl, 3-, 4- oder 5-Isoxazolyl, 5-Benzo[c]-isoxazolyl, 3-Benzo[d]isoxazolyl, 2-, 4- oder 5-Thiazolyl, 2-Benzothiazolyl, 3-, 4- oder 5-Isothiazolyl, 5-Benzo[c]isothiazolyl, 3-Benzo[d]isothiazolyl, 1-[($C_1$-$C_4$)Alkyl]-2-, -4- oder -5-imidazolyl, 1-[($C_1$-$C_4$)Alkyl]-2-benzimidazolyl, 1-[($C_1$-$C_4$)Alkyl]-3-, -4- oder -5-pyrazolyl, 2-[($C_1$-$C_4$)Alkyl]-3(2H)-indazolyl oder 1-[($C_1$-$C_4$)Alkyl]-3(1H)-indazolyl ist; oder eine dieser Gruppen ist am Kohlenstoff mit dem gleichen oder unterschiedlichen Substituenten, die Brom, Chlor, Fluor, ($C_1$-$C_4$)Alkyl, Trifluormethyl, Hydroxy, Hydroxymethyl oder ($C_1$-$C_4$)Alkoxy sind, mono- oder disubstituiert oder an benachbarten Kohlenstoffen mit Trimethylen, Tetramethylen, $-CH_2-0-CH_2-$ oder $-O-CH_2-0$ substituiert; und

$R^1$ ($C_1$-$C_8$)Alkyl, ($C_3$-$C_8$)Cycloalkyl, ($C_7$-$C_{10}$)Bicycloalkyl, ($C_4$-$C_{10}$)Cycloalkylalkyl, ($C_8$-$C_{11}$)-Bicycloalkylalkyl ist, oder eine dieser Gruppen ist mit der gleichen oder unterschiedlichen Gruppen, die Fluor, ($C_1$-$C_4$)Alkyl, ($C_1$-$C_4$)Alkoxy, Carboxy oder [($C_1$-$C_4$)Alkoxy]-carbonyl sind, mono- oder disubstituiert;

oder eines pharmazeutisch annehmbaren Säureadditionssalzes derselben oder

eines pharmazeutisch annehmbaren Kationensalzes, wenn die Verbindung eine Carboxygruppe enthält, das umfaßt:

(a) Umsetzung einer Verbindung der Formel

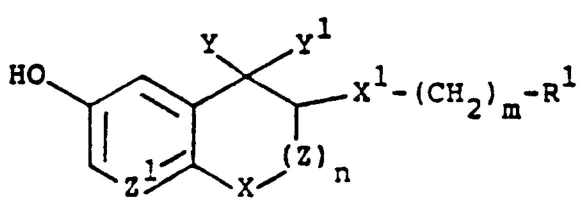

mit einer Verbindung der Formel

$R-CH_2-X^2$

worin $X^2$ eine nukleophil ersetzbare Gruppe ist, in Gegenwart einer Base;

(b) wenn Y und $Y^1$ getrennt genommen werden und Y Wasserstoff und $Y^1$ Hydroxy ist, Reduktion einer vorgebildeten Verbindung der Formel (I), worin Y und $Y^1$ zusammen genommen werden und eine Carbonylgruppe bilden;

(c) wenn Y und $Y^1$ zusammen genommen werden und eine Carbonylgruppe bilden und $X^1$ O oder S ist,

(i) Umsetzung einer Verbindung der Formel

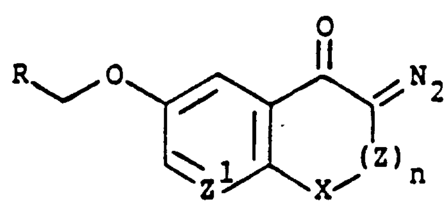

mit einer Verbindung der Formel

$R^1(CH_2)_mSH$ oder $R^1(CH_2)_mOH$

in Gegenwart von Rhodium(II)acetat-dimer;

oder

(ii) Umsetzung einer Verbindung der Formel

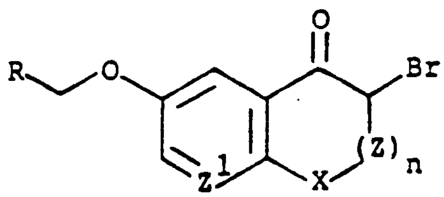

mit einer Verbindung der Formel

$R^1(CH_2)_mSH$ oder $R^1(CH_2)_mOH$

in Gegenwart einer Base;

(d) wenn Y und $Y^1$ zusammen genommen werden und eine Carbonylgruppe bilden und $X^1$ $CH_2$ ist, Hydrierung einer Verbindung der Formel

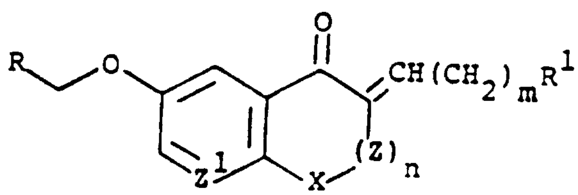

oder

(e) wenn $X^1$ SO oder $SO_2$ ist, Oxidation einer vorgebildeten Verbindung der Formel (I), worin $X^1$ S ist;

(f) wenn Y und $Y^1$ getrennt genommen werden und Y Wasserstoff und $Y^1$ eine Acyloxygruppe ist, Acylierung einer vorgebildeten Verbindung der Formel (I), worin Y und $Y^1$ getrennt genommen werden und Y Wasserstoff und $Y^1$ Hydroxy ist, und, falls gewünscht,

(g) Umwandeln einer vorgebildeten Verbindung der Formel (I) in ein pharmazeutisch annehmbares Säureadditionssalz oder, wenn sie eine Carboxygruppe enthält, in ein pharmazeutisch annehmbares Kationensalz.

**2.** Verfahren nach Anspruch 1 für eine Verbindung, in welcher Y und $Y^1$ zusammen genommen werden und eine Carbonylgruppe bilden.

**3.** Verfahren nach Anspruch 1 für eine Verbindung, worin Y und $Y^1$ getrennt genommen werden und Y Wasserstoff und $Y^1$ eine Acyloxygruppe ist, in welcher der Acylteil der $\alpha$-Aminoacylrest einer natürlich vorkommenden L-$\alpha$-Aminosäure,

$$-\overset{\overset{O}{\|}}{C}-(CH_2)_pNR^2R^3,\quad -\overset{\overset{O}{\|}}{C}-CHNH_2(CH_2)_qNR^2R^3,\quad -\overset{\overset{O}{\|}}{C}-(CH_2)_rCOOH \text{ oder}$$

$$-\overset{\overset{O}{\|}}{C}-CHNH_2(CH_2)_sCOOH$$

ist; $R^2$ und $R^3$ getrennt genommen werden und jeweils unabhängig Wasserstoff oder $(C_1\text{-}C_4)$Alkyl sind, oder $R^2$ und $R^3$ zusammen mit dem Stickstoffatom, an welches sie gebunden sind, genommen werden und einen Pyrrolidin-, Piperidin-, Perhydroazepin- oder Morpholinring bilden;
p eine ganze Zahl von 1 bis 4 ist;
q eine ganze Zahl von 1 bis 3 ist;

r eine ganze Zahl von 2 bis 3 ist; und
s eine ganze Zahl von 1 bis 3 ist.

**4.** Verfahren nach Anspruch 1 für eine Verbindung, worin Y und $Y^1$ getrennt genommen werden und Y Wasserstoff und $Y^1$ Hydroxy ist.

**5.** Verfahren nach Anspruch 1, worin n 1 ist, Z $CH_2$ ist, $Z^1$ CH ist, X O ist, $X^1$ $CH_2$ oder O ist, R 2-Pyridyl oder 2-Chinolyl ist und $R^1$ ($C_2$-$C_8$)Alkyl oder ($C_3$-$C_8$)-Cycloalkyl ist.

**6.** Verfahren nach Anspruch 5 für eine racemische oder optisch aktive Verbindung mit der relativen stereochemischen Formel

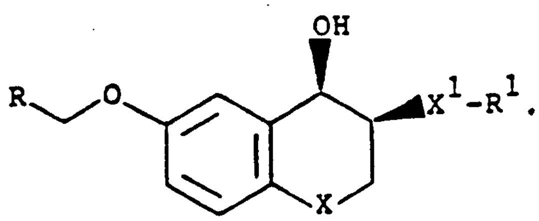

**7.** Verfahren nach Anspruch 6 für eine Verbindung, worin X O ist, $X^1$ O oder $CH_2$ ist, R 2-Pyridyl oder 2-Chinoyl ist und $R^1$ Propyl, Isopropyl oder Cyclohexyl ist.

**8.** Verfahren nach Anspruch 5 für eine racemische oder optisch aktive Verbindung mit der relativen stereochemischen Formel

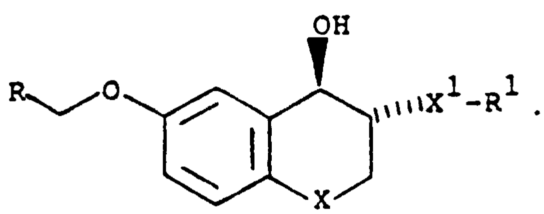

**9.** Verfahren nach Anspruch 8 für eine Verbindung, worin X O ist, $X^1$ O oder $CH_2$ ist, R 2-Pyridyl oder 2-Chinolyl ist und $R^1$ Propyl, Isopropyl oder Cyclohexyl ist.

**10.** Verbindung mit der Formel

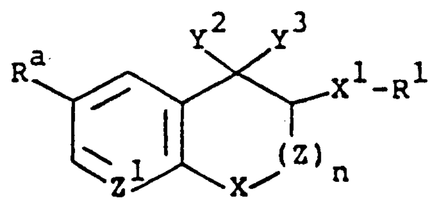

worin

$R^a$ Hydroxy oder Benzyloxy ist,
n = 0 oder 1 ist,
X O, S, SO, $SO_2$, NH oder N($C_1$-$C_4$)Alkyl ist,
$X^1$ $CH_2$, O, S, SO oder $SO_2$ ist,

$Z^1$ CH oder N ist,

$Y^2$ und $Y^3$ zusammen genommen werden und Carbonyl sind oder $Y^2$ und $Y^3$ getrennt genommen werden und $Y^2$ Wasserstoff und $Y^3$ Hydroxy ist und

$R^1$ $(C_1-C_8)$Alkyl, $(C_3-C_8)$Cycloalkyl, $(C_7-C_{10})$Bicycloalkyl, $(C_4-C_{10})$Cycloalkylalkyl, $(C_8-C_{11})$-Bicycloalkylalkyl oder eine dieser Gruppen ist, die mit der gleichen oder unterschiedlichen Gruppen, die Fluor, $(C_1-C_4)$Alkyl, $(C_1-C_4)$Alkoxy, Carboxy oder $[(C_1-C_4)$Alkoxy]carbonyl sind, mono- oder disubstituiert ist.

**Revendications**

**Revendications pour les Etats contractants suivants : AT, BE, CH, DE, FR, GB, IT, LI, LU, NI, SE**

**1.** Composé racémique ou optiquement actif, répondant à la formule structurale

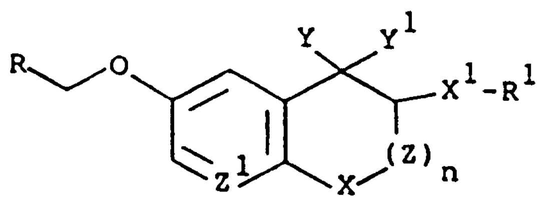

dans laquelle

n a la valeur 0 ou 1 ;

X représente O, S, SO, $SO_2$, NH ou N(alkyle en $C_1$ à $C_4$) ;

$X^1$ représente $CH_2$, O, S, SO ou $SO_2$ ;

Y et $Y^1$ sont considérés ensemble et forment un groupe carbonyle, ou bien Y et $Y^1$ sont considérés séparément, auquel cas Y représente l'hydrogène et $Y^1$ est un groupe hydroxy ou un groupe acyloxy qui est hydrolysé en formant un groupe hydroxy dans des conditions physiologiques ;

Z est un groupe $CH_2$, $CHCH_3$, $CH_2CH_2$ ou $CH_2CH_2CH_2$ ;

$Z^1$ représente CH ou N ;

R est un groupe 2-, 3- ou 4-pyridyle, 2-, 3- ou 4-quinolyle, 1-, 3- ou 4-isoquinolyle, 3- ou 4-pyridazinyle, 3- ou 4-cinnolinyle, 1-phtalazinyle, 2- ou 4-pyrimidinyle, 2- ou 4-quinazolinyle, 2-pyrazinyle, 2-quinoxalinyle, 1-, 2- ou 3-indolizinyle, 2-, 4- ou 5-oxazolyle, 2-benzoxazolyle, 3-, 4- ou 5-isoxazolyle, 5-benzo[c]isoxazolyle, 3-benzo[d]isoxazolyle, 2-, 4- ou 5-thiazolyle, 2-benzothiazolyle, 3-, 4- ou 5-isothiazolyle, 5-benzo [c] isothiazolyle, 3-benzo[d]isothiazolyle, 1-(alkyle en $C_1$ à $C_4$)-2-, 4- ou 5-imidazolyle, 1-(alkyle en $C_1$ à $C_4$)-2-benzimidazolyle, 1-(alkyle en $C_1$ à $C_4$)-3-, 4- ou 5-pyrazolyle, 2-(alkyle en $C_1-C_4$)-3(2H)-indazolyle, ou 1-(alkyle en $C_1-C_4$)-3(1H)-indazolyle ; ou bien l'un desdits groupes monosubstitué ou disubstitué sur du carbone avec les mêmes substituants ou des substituants différents qui sont des radicaux bromo, chloro, fluoro, alkyle en $C_1$ à $C_4$, trifluorométhyle, hydroxy, hydroxyméthyle, ou alkoxy en $C_1$ à $C_4$, ou substitué sur des atomes adjacents de carbone avec un groupe triméthylène, tétraméthylène, $-CH_2-O-CH_2-$ ou $-O-CH_2-O$ ; et

$R^1$ est un groupe alkyle en $C_1$ à $C_8$, cycloalkyle en $C_3$ à $C_8$, bicycloalkyle en $C_7$ à $C_{10}$, cycloalkylalkyle en $C_4$ à $C_{10}$, bicycloalkylalkyle en $C_8$ à $C_{11}$ ou bien l'un desdits groupes monosubstitué ou disubstitué avec le même groupe ou des groupes différents qui sont des radicaux fluoro, alkyle en $C_1$ à $C_4$, alkoxy en $C_1$ à $C_4$, carboxy ou (alkoxy en $C_1$ à $C_4$)- carbonyle ;

un sel d'addition d'acide pharmaceutiquement acceptable de ce composé ; ou

un sel cationique acceptable du point de vue pharmaceutique lorsque le composé contient un groupe carboxy ;

**2.** Composé suivant la revendication 1, dans lequel Y et $Y^1$ sont considérés ensemble et forment un groupe carbonyle.

**3.** Composé suivant la revendication 1, dans lequel Y et $Y^1$ sont considérés séparément, Y est l'hydrogène et $Y^1$ est un groupe acyloxy dont la partie acyle est le résidu alpha-aminoacyle d'un L-alpha-aminoacide naturel,

$-\overset{O}{\overset{\|}{C}}-(CH_2)_pNR^2R^3$, $-\overset{O}{\overset{\|}{C}}-CHNH_2(CH_2)_qNR^2R^3$, $-\overset{O}{\overset{\|}{C}}-(CH_2)_rCOOH$, ou

$-\overset{O}{\overset{\|}{C}}-CHNH_2(CH_2)_sCOOH$;

$R^2$ et $R^3$ sont considérés séparement et représentent chacun, indépendemment, l'hydrogène ou un groupe alkyle en $C_1$ à $C_4$, ou bien $R^2$ et $R^3$ forment conjointement avec l'atome d'azote auquel ils sont attachés, un noyau de pyrrolidine, piperidine, perhydroazépine ou morpholine ;

p est un nombre entier de 1 à 4 ;

q est un nombre entier de 1 à 3 ;

r est le nombre entier 2 ou 3 ;

s est un nombre entier de 1 à 3.

**4.** Composé suivant la revendication 1, dans lequel Y et $Y^1$ sont considérés séparément, Y étant l'hydrogène et $Y^1$ étant un groupe hydroxy.

**5.** Composé suivant la revendication 4, dans lequel n est égal à 1, Z est un groupe $CH_2$, $Z^1$ est un groupe CH, X et $X^1$ représentent chacun, indépendamment, $CH_2$ ou O, R est un groupe 2-pyridyle ou 2-quinolyle et $R^1$ est un groupe alkyle en $C_2$ à $C_8$ ou cycloalkyle en $C_3$ à $C_8$.

**6.** Composé racémique ou optiquement actif suivant la revendication 5, répondant à la formule stéréochimique relative

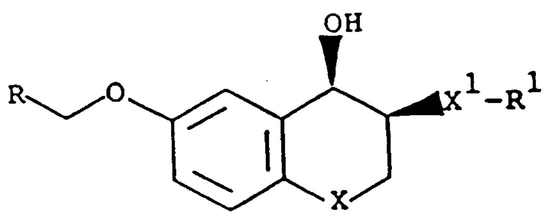

.

**7.** Composé suivant la revendication 6, dans lequel X représente O, $X^1$ représente O ou $CH_2$, R est un groupe 2-pyridyle ou 2-quinolyle et $R^1$ est un groupe propyle, isopropyle ou cyclohexyle.

**8.** Composé racémique ou optiquement actif suivant la revendication 5, répondant à la formule stéréochimique relative

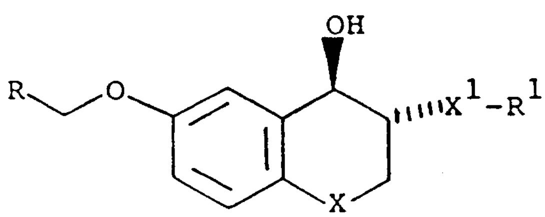

.

**9.** Composé suivant la revendication 8, dans lequel X représente O, $X^1$ représente O ou $CH_2$, R est un groupe 2-pyridyle ou 2-quinolyle et $R^1$ est un groupe propyle, isopropyle ou cyclohexyle.

**10.** Composé de formule

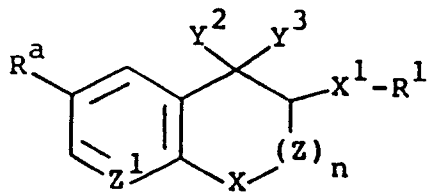

dans laquelle

$R^a$ est un groupe hydroxy ou benzyloxy ;

n a la valeur 0 ou 1 ;

X représente O, S, SO, $SO_2$, NH ou N-(alkyle en $C_1$ à $C_4$) ;

$X^1$ représente $CH_2$, O, S, SO ou $SO_2$ ;

$Z^1$ représente CH ou N ;

$Y^2$ et $Y^3$ sont considérés ensemble et forment un groupe carbonyle ou bien $Y^2$ et $Y^3$ sont considérés séparément, $Y^2$ est l'hydrogène et $Y^3$ est un groupe hydroxy ; et

$R^1$ est un groupe alkyle en $C_1$ à $C_8$, cycloalkyle en $C_3$ à $C_8$, bicycloalkyle en $C_7$ à $C_{10}$, cycloalkylalkyle en $C_4$-$C_{10}$, bicycloalkylalkyle en $C_8$ à $C_{11}$, ou bien l'un desdits groupes monosubstitué ou disubstitué avec le même groupe ou des groupes différents qui sont des radicaux fluoro, alkyle en $C_1$ à $C_4$, alkoxy en $C_1$ à $C_4$, carboxy ou (alkoxy en $C_1$ à $C_4$)-carbonyle.

**11.** Composition pharmaceutique destinée à être administrée à un mamifère, qui comprend une quantité inhibitrice de 5-lipoxygénase et/ou une quantité à effet de blocage des récepteurs D4 de leukotriène d'un composé suivant la revendication 1 et un support acceptable du point de vue pharmaceutique.

**12.** Compose suivant l'une quelconque des revendications 1 à 9 destiné à inhiber l'enzyme 5-lipoxygénase et/ou à bloquer les récepteurs de leukotriène.

**13.** Utilisation d'un composé suivant l'une quelconque des revendications 1 à 9 pour la préparation d'un médicament destiné à inhiber l'enzyme 5-lipoxygénase et/ou à bloquer les récepteurs de leukotriène.

**Revendications pour l'Etat contractant suivant : ES**

**1.** Procédé de production d'un composé racémique ou optiquement actif de formule structurale

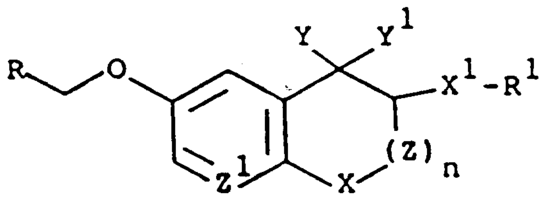

dans laquelle

n a la valeur 0 ou 1 ;

X représente O, S, SO, $SO_2$, NH ou N(alkyle en $C_1$ à $C_4$) ;

$X^1$ représente $CH_2$, O, S, SO ou $SO_2$ ;

Y et $Y^1$ sont considérés ensemble et forment un groupe carbonyle, ou bien Y et $Y^1$ sont considérés séparément, auquel cas Y représente l'hydrogène et $Y^1$ est un groupe hydroxy ou un groupe acyloxy qui est hydrolysé en formant un groupe hydroxy dans des conditions physiologiques ;

Z est un groupe $CH_2$, $CHCH_3$, $CH_2CH_2$ ou $CH_2CH_2CH_2$ ;

$Z^1$ représente CH ou N ;

R est un groupe 2-, 3- ou 4-pyridyle, 2-, 3- ou 4-quinolyle, 1-, 3- ou 4-isoquinolyle, 3- ou 4-pyridazinyle, 3- ou 4-cinnolinyle, 1-phtalazinyle, 2- ou 4-pyrimidinyle, 2- ou 4-quinazolinyle, 2-pyrazinyle, 2-quinoxalinyle, 1-, 2- ou 3-indolizinyle, 2-, 4- ou 5-oxazolyle, 2-benzoxazolyle, 3-, 4- ou 5-isoxazolyle, 5-benzo[c]isoxazolyle, 3-benzo[d]isoxazolyle, 2-, 4- ou 5-thiazolyle, 2-benzothiazolyle, 3-, 4-

ou 5-isothiazolyle, 5-benzo[c]isothiazolyle, 3-benzo[d]isothiazolyle, 1-(alkyle en $C_1$ à $C_4$)-2-, 4- ou 5-imidazolyle, 1-(alkyle en $C_1$ à $C_4$)-2-benzimidazolyle, 1-(alkyle en $C_1$ à $C_4$)-3-, 4- ou 5-pyrazolyle, 2-(alkyle en $C_1$ à $C_4$)-3(2H)-indazolyle ou 1-(alkyle en $C_1$ à $C_4$)-3(1H)-indazolyle ; ou bien l'un desdits groupes monosubstitué ou disubstitué sur du carbone avec les mêmes substituants ou des substituants différents qui sont des radicaux bromo, chloro, fluoro, alkyle en $C_1$ à $C_4$, trifluorométhyle, hydroxy, hydroxyméthyle, ou alkoxy en $C_1$ à $C_4$, ou substitué sur des atomes adjacents de carbone avec un groupe triméthylène, tétraméthylène, $-CH_2-O-CH_2-$ ou $-O-CH_2-O$ ; et

$R^1$ est un groupe alkyle en $C_1$ à $C_8$, cycloalkyle en $C_3$ à $C_8$, bicycloalkyle en $C_7$ à $C_{10}$, cycloalkylalkyle en $C_4$ à $C_{10}$, bicycloalkylalkyle en $C_8$ à $C_{11}$, ou bien l'un desdits groupes monosubstitué ou disubstitué avec le même groupe ou des groupes différents qui sont des radicaux fluoro, alkyle en $C_1$ à $C_4$, alkoxy en $C_1$ à $C_4$ carboxy ou (alkoxy en $C_1$ à $C_4$)-carbonyle ;

d'un sel d'addition d'acide pharmaceutiquement acceptable de ce composé ; ou

d'un sel cationique acceptable du point de vue pharmaceutique lorsque le composé contient un groupe carboxy ;

qui comprend :

(a) la réaction d'un composé de formule

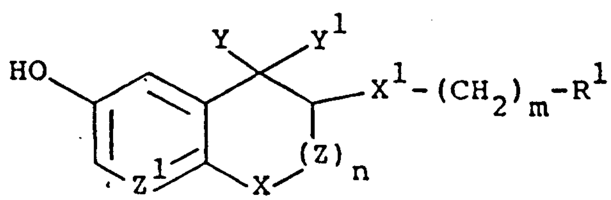

avec un composé de formule

$R-CH_2-X^2$

dans laquelle $X^2$ est un groupe susceptible de déplacement nucléophile, en présence d'une base ;

(b) lorsque Y et $Y^1$ sont considérés séparément, Y est l'hydrogène et $Y^1$ est un groupe hydroxy, la réduction d'un composé préformé de formule (I) dans laquelle Y et $Y^1$ sont considérés ensemble et forment un groupe carbonyle ;

(c) lorsque Y et $Y^1$ sont considérés ensemble et forment un groupe carbonyle et $X^1$ représente O ou S,

(i) la réaction d'un composé de formule

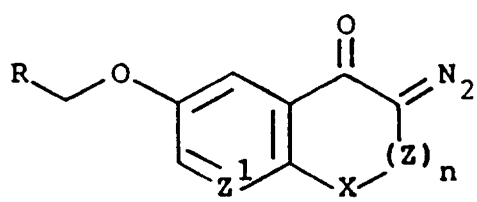

avec un composé de formule

$R^1(CH_2)_mSH$ ou $R^1(CH_2)_mOH$

en présence de dimère d'acétate de rhodium (II) ; ou bien

(ii) la réaction d'un composé de formule :

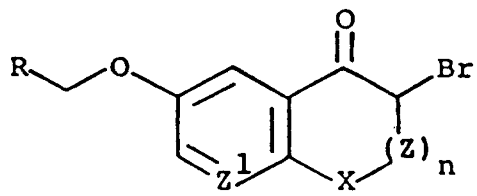

avec un composé de formule

$R^1(CH_2)_mSH$ ou $R^1(CH_2)_mOH$

en présence d'une base ;

(d) lorsque Y et $Y^1$ sont considérés ensemble et forment un groupe carbonyle et $X^1$ est un groupe $CH_2$, l'hydrogénation d'un composé de formule :

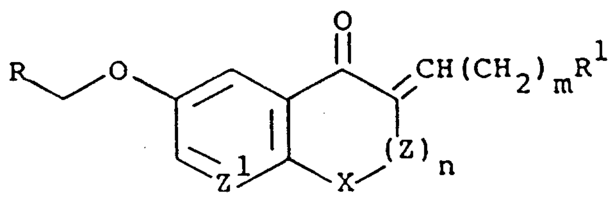

;

ou

(e) lorsque $X^1$ représente SO ou $SO_2$, l'oxydation d'un composé préformé de formule (I) dans laquelle $X^1$ représente S ;

(f) lorsque Y et $Y^1$ sont considérés séparément, Y est l'hydrogène et $Y^1$ est un groupe acyloxy, l'acylation d'un composé préformé de formule (I) dans laquelle Y et $Y^1$ sont considérés séparément, Y est l'hydrogène et $Y^1$ est un groupe hydroxy ; et, le cas échéant,

(g) la transformation d'un composé préformé de formule (I) en un sel d'addition d'acide pharmaceutiquement acceptable, ou bien, lorsqu'il contient un groupe carboxy, en un sel cationique pharmaceutiquement acceptable.

**2.** Procédé suivant la revendication 1, destiné à un composé dans lequel Y et $Y^1$ sont considérés ensemble et forment un groupe carbonyle.

**3.** Procédé suivant la revendication 1, destiné à un composé dans lequel Y et $Y^1$ sont considérés séparément, Y est l'hydrogène et $Y^1$ est un groupe acyloxy dont la portion acyle est le résidu alpha-aminoacyle d'un L-alpha-aminoacide naturel,

$$-\overset{O}{\overset{\|}{C}}-(CH_2)_pNR^2R^3, \quad -\overset{O}{\overset{\|}{C}}-CHNH_2(CH_2)_qNR^2R^3, \quad -\overset{O}{\overset{\|}{C}}-(CH_2)_rCOOH, \text{ ou}$$

$$-\overset{O}{\overset{\|}{C}}-CHNH_2(CH_2)_sCOOH;$$

$R^2$ et $R^3$ sont considérés séparément et représentent chacun, indépendamment, l'hydrogène ou un groupe alkyle en $C_1$ à $C_4$, ou bien $R^2$ et $R^3$ forment conjointement avec l'atome d'azote auquel ils sont attachés, un noyau de pyrrolidine, piperidine, perhydroazépine ou morpholine ;

p est un nombre entier de 1 à 4

q est un nombre entier de 1 à 3

r est le nombre entier 2 ou 3 ; et
s est un nombre entier de 1 à 3.

4. Procédé suivant la revendication 1, pour l'obtention d'un composé dans lequel Y et $Y^1$ sont considérés séparément, Y est l'hydrogène et $Y^1$ est un groupe hydroxy.

5. Procédé suivant la revendication 4, dans lequel n est égal à 1, Z représente $CH_2$, $Z^1$ est un groupe CH, X représente O, $X^1$ représente $CH_2$ ou O, R est un groupe 2-pyridyle ou 2-quinolyle et $R^1$ est un groupe alkyle en $C_2$ à $C_8$ ou cycloalkyle en $C_3$ à $C_8$.

6. Procédé suivant la revendication 5, pour l'obtention d'un composé racémique ou optiquement actif répondant à la formule stéréochimique relative

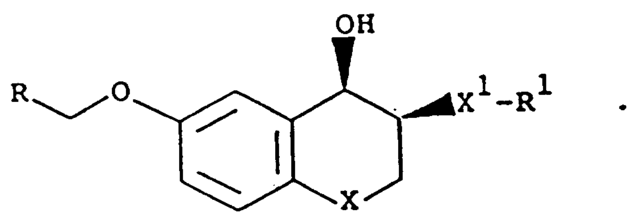

7. Procédé suivant la revendication 6, pour l'obtention d'un composé dans lequel X représente O, $X^1$ représente O ou $CH_2$, R est un groupe 2-pyridyle ou 2-quinolyle et $R^1$ est un groupe propyle, isopropyle ou cyclohexyle.

8. Procédé suivant la revendication 5, pour l'obtention d'un composé racémique ou optiquement actif répondant à la formule stéréochimique relative

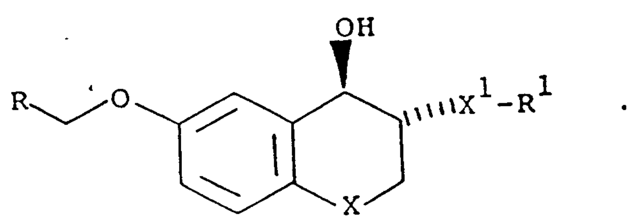

9. Procédé suivant la revendication 8, pour l'obtention d'un composé dans lequel X représente O, $X^1$ représente O ou $CH_2$, R est un groupe 2-pyridyle ou 2-quinolyle et $R^1$ est un groupe propyle, isopropyle ou cyclohexyle.

**Revendications pour l'Etat contractant suivant : GR**

1. Procédé de production d'un composé racémique ou optiquement actif de formule structurale

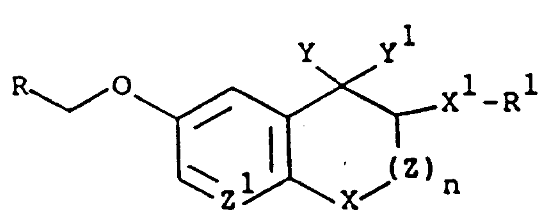

dans laquelle
n a la valeur 0 ou 1 ;

X représente O, S, SO, $SO_2$, NH ou N(alkyle en $C_1$ à $C_4$) ;

$X^1$ représente $CH_2$, O, S, SO ou $SO_2$ ;

Y et $Y^1$ sont considérés ensemble et forment un groupe carbonyle, ou bien Y et $Y^1$ sont considérés séparément, auquel cas Y représente l'hydrogène et $Y^1$ est un groupe hydroxy ou un groupe acyloxy qui est hydrolysé en formant un groupe hydroxy dans des conditions physiologiques ;

Z est un groupe $CH_2$, $CHCH_3$, $CH_2CH_2$ ou $CH_2CH_2CH_2$ ;

$Z^1$ représente CH ou N ;

R est un groupe 2-, 3- ou 4-pyridyle, 2-, 3- ou 4-quinolyle, 1-, 3- ou 4-isoquinolyle, 3- ou 4-pyridazinyle, 3- ou 4-cinnolinyle, 1-phtalazinyle, 2- ou 4-pyrimidinyle, 2- ou 4-quinazolinyle, 2-pyrazinyle, 2-quinoxalinyle, 1-, 2- ou 3-indolizinyle, 2-, 4- ou 5-oxazolyle, 2-benzoxazolyle, 3-, 4- ou 5-isoxazolyle, 5-benzo[c]isoxazolyle, 3-benzo[d]isoxazolyle, 2-, 4- ou 5-thiazolyle, 2-benzothiazolyle, 3-, 4- ou 5-isothiazolyle, 5-benzo[c]isothiazolyle, 3-benzo[d]isothiazolyle, 1-(alkyle en $C_1$ à $C_4$)-2-, 4- ou 5-imidazolyle, 1-(alkyle en $C_1$ à $C_4$)-2-benzimidazolyle, 1-(alkyle en $C_1$ à $C_4$)-3-, 4- ou 5-pyrazolyle, 2-(alkyle en $C_1$ à $C_4$)-3(2H)-indazolyle, ou 1-(alkyle en $C_1$ à $C_4$)-3(1H)-indazolyle ; ou bien l'un desdits groupes monosubstitué ou disubstitué sur du carbone avec les mêmes substituants ou des substituants différents qui sont des radicaux bromo, chloro, fluoro, alkyle en $C_1$ à $C_4$, trifluorométhyle, hydroxy, hydroxyméthyle ou alkoxy en $C_1$ à $C_4$, ou substitué sur des atomes adjacents de carbone avec un groupe triméthylène, tétraméthylène, $-CH_2-O-CH_2-$ ou $-O-CH_2-O$ ; et

$R^1$ est un groupe alkyle en $C_1$ à $C_8$, cycloalkyle en $C_3$ à $C_8$, bicycloalkyle en $C_7$ à $C_{10}$, cycloalkylalkyle en $C_4$ à $C_{10}$, bicycloalkylalkyle en $C_8$ à $C_{11}$ ou bien l'un desdits groupes monosubstitué ou disubstitué avec le même groupe ou des groupes différents qui sont des radicaux fluoro, alkyle en $C_1$ à $C_4$, alkoxy en $C_1$ à $C_4$, carboxy ou (alkoxy en $C_1$ à $C_4$)-carbonyle ;

d'un sel d'addition d'acide pharmaceutiquement acceptable de ce composé ; ou

d'un sel cationique acceptable du point de vue pharmaceutique lorsque le composé contient un groupe carboxy ;

qui comprend :

(a) la réaction d'un composé de formule

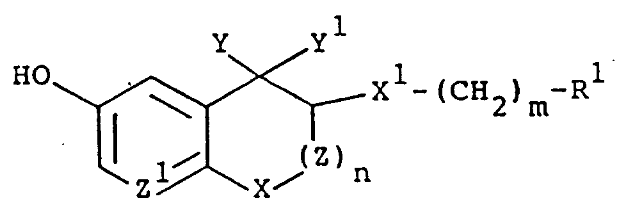

avec un composé de formule

$R-CH_2-X^2$

dans laquelle $X^2$ est un groupe susceptible de déplacement nucléophile, en présence d'une base ;

(b) lorsque Y et $Y^1$ sont considérés séparément, Y est l'hydrogène et $Y^1$ est un groupe hydroxy, la réduction d'un composé préformé de formule (I) dans laquelle Y et $Y^1$ sont considérés ensemble et forment un groupe carbonyle ;

(c) lorsque Y et $Y^1$ sont considérés ensemble et forment un groupe carbonyle et $X^1$ représente O ou S,

(i) la réaction d'un composé de formule

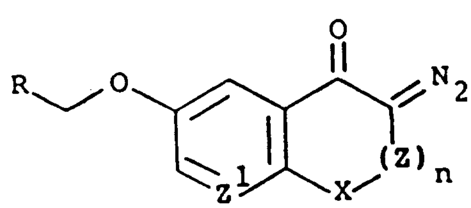

avec un composé de formule

$R^1(CH_2)_mSH$ ou $R^1(CH_2)_mOH$

en présence de dimère d'acétate de rhodium (II) ; ou bien

(ii) la réaction d'un composé de formule :

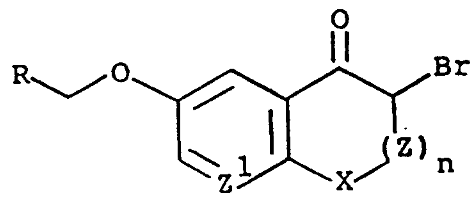

avec un composé de formule

$R^1(CH_2)_mSH$ ou $R^1(CH_2)_mOH$

en présence d'une base ;

(d) lorsque Y et $Y^1$ sont considérés ensemble et forment un groupe carbonyle et $X^1$ est un groupe $CH_2$, l'hydrogénation d'un composé de formule :

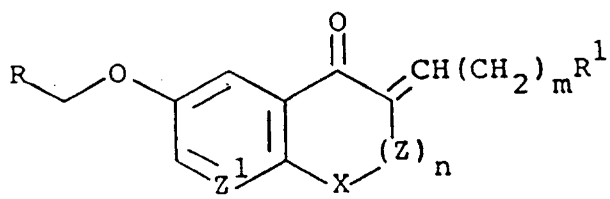 ;

ou

(e) lorsque $X^1$ représente SO ou $SO_2$, l'oxydation d'un composé préformé de formule (I) dans laquelle $X^1$ représente S ;

(f) lorsque Y et $Y^1$ sont considérés séparément, Y est l'hydrogène et $Y^1$ est un groupe acyloxy, l'acylation d'un composé préformé de formule (I) dans laquelle Y et $Y^1$ sont considérés séparément, Y est l'hydrogène et $Y^1$ est un groupe hydroxy ; et, le cas échéant,

(g) la transformation d'un composé préformé de formule (I) en un sel d'addition d'acide pharmaceutiquement acceptable, ou bien, lorsqu'il contient un groupe carboxy, en un sel cationique pharmaceutiquement acceptable.

**2.** Procédé suivant la revendication 1, destiné à un composé dans lequel Y et $Y^1$ sont considérés ensemble et forment un groupe carbonyle.

**3.** Procédé suivant la revendication 1, destiné à un composé dans lequel Y et $Y^1$ sont considérés séparément, Y est l'hydrogène et $Y_1$ est un groupe alcyloxy dont la portion acyle est le résidu alpha-aminoacyle d'un L-alpha-amino acide naturel,

$$-\overset{\overset{O}{\|}}{C}-(CH_2)_pNR^2R^3,\quad -\overset{\overset{O}{\|}}{C}-CHNH_2(CH_2)_qNR^2R^3,\quad -\overset{\overset{O}{\|}}{C}-(CH_2)_rCOOH,\ \text{ou}$$

$$-\overset{\overset{O}{\|}}{C}-CHNH_2(CH_2)_sCOOH;$$

$R^2$ et $R^3$ sont considérés séparément et représentent chacun, indépendamment, l'hydrogène ou un groupe alkyle en $C_1$ à $C_4$, ou bien $R^2$ et $R^3$ forment conjointement avec l'atome d'azote auquel ils sont attachés un noyau de pyrrolidine, piperidine, perhydroazépine ou morpholine ;

p est un nombre entier de 1 à 4 ;

q est un nombre entier de 1 à 3 ;

r est le nombre entier 2 ou 3 ; et

s est un nombre entier de 1 à 3.

**4.** Procédé suivant la revendication 1, pour l'obtention d'un composé dans lequel Y et $Y^1$ sont considérés séparément, Y est l'hydrogène et $Y^1$ est un groupe hydroxy.

**5.** Procédé suivant la revendication 4, dans lequel n est égal à 1, Z représente $CH_2$, $Z^1$ est un groupe CH, X représente O, $X^1$ représente $CH_2$ ou O, R est un groupe 2-pyridyle ou 2-quinolyle et $R^1$ est un groupe alkyle en $C_2$ à $C_8$ ou cycloalkyle en $C_3$ à $C_8$.

**6.** Procédé suivant la revendication 5, pour l'obtention d'un composé racémique ou optiquement actif répondant à la formule stéréochimique relative

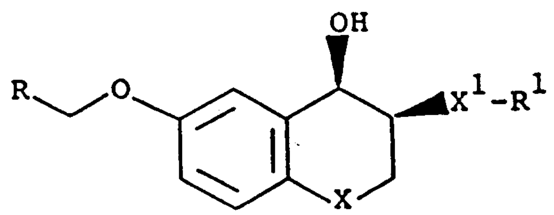 .

**7.** Procédé suivant la revendication 6, pour l'obtention d'un composé dans lequel X représente O, $X^1$ représente O ou $CH_2$, R est un groupe 2-pyridyle ou 2-quinolyle et $R^1$ est un groupe propyle, isopropyle ou cyclohexyle.

**8.** Procédé suivant la revendication 5, pour l'obtention d'un composé racémique ou optiquement actif répondant à la formule stéréochimique relative

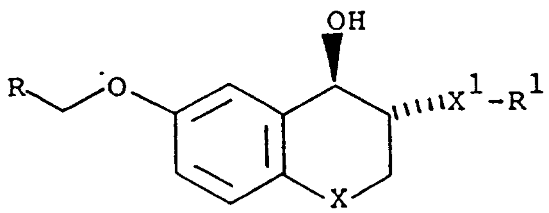 .

**9.** Procédé suivant la revendication 8, pour l'obtention d'un composé dans lequel X représente O, $X^1$ représente O ou $CH_2$, R est un groupe 2-pyridyle ou 2-quinolyle et $R^1$ est un groupe propyle, isopropyle ou cyclohexyle.

**10.** Composé de formule

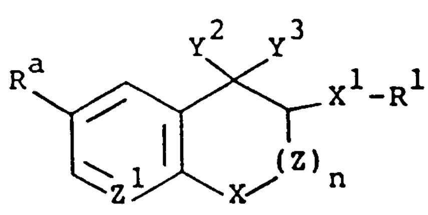

dans laquelle

$R^a$ est un groupe hydroxy ou benzyloxy ;

n a la valeur 0 ou 1 ;

X représente O, S, SO, $SO_2$, NH ou N-(alkyle en $C_1$ à $C_4$) ;

$X^1$ représente $CH_2$, O, S, SO ou $SO_2$ ;

$Z^1$ représente CH ou N ;

$Y^2$ et $Y^3$ sont considérés ensemble et forment un groupe carbonyle, ou bien $Y^2$ et $Y^3$ sont considérés séparément, $Y^2$ est l'hydrogène et $Y^3$ est un groupe hydroxy ; et

$R^1$ est un groupe alkyle en $C_1$ à $C_8$, cycloalkyle en $C_3$ à $C_8$, bicycloalkyle en $C_7$ à $C_{10}$, cycloalkylalkyle en $C_4$ à $C_{10}$, bicycloalkylalkyle en $C_8$ à $C_{11}$, ou bien l'un desdits groupes monosubstitués ou disubstitués avec le même groupe ou des groupes différents qui sont des radicaux fluoro, alkyle en $C_1$ à $C_4$, alkoxy en $C_1$ à $C_4$, carboxy ou (alkoxy en $C_1$ à $C_4$)-carbonyle.